(19) Europäisches Patentamt / European Patent Office / Office européen des brevets

(11) EP 3 369 749 A1

(12) **EUROPEAN PATENT APPLICATION**
published in accordance with Art. 153(4) EPC

(43) Date of publication:
05.09.2018 Bulletin 2018/36

(51) Int Cl.:
*C08B 11/02* (2006.01)

(21) Application number: 16859855.5

(22) Date of filing: 26.10.2016

(86) International application number:
PCT/JP2016/081748

(87) International publication number:
WO 2017/073626 (04.05.2017 Gazette 2017/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 30.10.2015 JP 2015214372
05.02.2016 JP 2016020548
05.02.2016 JP 2016020749
31.05.2016 JP 2016108946

(71) Applicant: Toray Industries, Inc.
Tokyo 103-8666 (JP)

(72) Inventors:
• OTSUBO, Takahiko
Nagoya-shi
Aichi 455-8502 (JP)
• OHTA, Kotoe
Kamakura-shi
Kanagawa 248-8555 (JP)

• ECHIGO, Yuji
Nagoya-shi
Aichi 455-8502 (JP)
• TAKEZAKI, Hiroshi
Nagoya-shi
Aichi 455-8502 (JP)
• TAKAKI, Suguru
Kamakura-shi
Kanagawa 248-8555 (JP)
• MINAKAMI, Satoshi
Kamakura-shi
Kanagawa 248-8555 (JP)
• ITO, Akinori
Mishima-shi
Shizuoka 411-8652 (JP)
• MIYAMA, Hisashi
Tokyo 103-8666 (JP)

(74) Representative: Webster, Jeremy Mark et al
Mewburn Ellis LLP
City Tower
40 Basinghall Street
London EC2V 5DE (GB)

(54) **CELLULOSE ETHER DERIVATIVE FINE PARTICLES**

(57)      In a system where phase separation into two phases occurs when an ether cellulose derivative (A), a polymer (B) different from the ether cellulose derivative (A), and an alcohol solvent (C) are mixed together, the two phases including a solution phase mainly containing the ether cellulose derivative (A) and a solution phase mainly containing the polymer (B), the two separated phases containing approximately the same solvent, an emulsion is formed and brought into contact with a poor solvent (D) to provide an ether cellulose derivative microparticle having an average particle diameter of 1 to 1,000 μm, a linseed oil absorption of 50 to 1,000 mL/100 g, and an average surface pore size of 0.05 to 5 μm.

EP 3 369 749 A1

**Description**

CROSS-REFERENCE TO RELATED APPLICATION

The present application claims priority to Japanese Patent Application No. 2015-214372 filed on October 30, 2015, Japanese Patent Application No. 2016-20548 and Japanese Patent Application No. 2016-20749 filed on February 5, 2016, and Japanese Patent Application No. 2016-108946 filed on May 31, 2016. The contents of these Japanese applications are entirely incorporated herein by reference.

Technical Field

The present invention relates to a porous ether cellulose derivative microparticle and a method for producing the same. Specifically, the present invention relates to a porous ether cellulose derivative microparticle having pores both on the surface of and inside the particle, and a method for producing the same.

Background Art

Microparticles having high specific surface areas can be widely used as carriers that support or adsorb target substances on the surface of the microparticles. Examples of specific applications include use as carriers that support substances exhibiting catalysis, adsorbents for target components existing in media, and carriers in columns in chromatography.

Examples of a method for increasing the specific surface areas of microparticles include making the microparticles porous. Making the microparticles porous not only increases the specific surface areas of the microparticles and increases supported amounts of target substances but also enables the target substances to be incorporated and retained in fine pores in the microparticles.

Known examples of the porous microparticles include porous silica microparticles. However, since inorganic microparticles such as silica microparticles have large specific gravities, the microparticles easily settle by gravitation in a medium containing a target substance and are easily separated from the medium. In the case where, for example, such microparticles are made to support a substance exhibiting catalysis and used in a chemical reaction, the microparticles settle and accumulate, which causes localization of the catalyst on the microparticles. Such a situation is not preferable. Also, when a target component existing in a medium is to be collected by adsorption on the microparticles, the difficulty in making the microparticles float in the medium reduces the number of contact with the target component and thus lowers adsorption efficiency, which is not a preferable situation.

Porous polymer microparticles are used in some cases as microparticles having specific gravities smaller than the specific gravities of inorganic microparticles. However, use of porous microparticles made of polymers derived from petroleum raw materials is not preferable in view of the environment, and substitution of porous microparticles made of polymers derived from nonpetroleum raw materials is desired.

Nonpetroleum polymers are carbon-neutral materials and are thus environment-friendly. Porous microparticles made of nonpetroleum polymers not only have the advantage that the specific gravities are small and functional features peculiar to porous shapes but also have the characteristic of imposing low environmental burdens. Development of microparticles made of cellulose and cellulose derivatives, which are nonpetroleum polymers, is under consideration.

For example, Patent Document 1 discloses a method for obtaining cellulose particles by forming a W/O/W emulsion of a cellulose fatty acid ester, distilling off the solvent in the organic phase to extract porous microparticles of the cellulose fatty acid ester, and saponifying the particles. In addition, as disclosed in Patent Document 2, there is a method for obtaining porous cellulose microparticles by dissolving cellulose in an ionic liquid, then adding the solution to a solvent that is not compatible with the ionic liquid to form an emulsion, and bringing the resulting emulsion into contact with an alcohol solvent. Also, Patent Document 3 and Patent Document 4 each disclose a method for obtaining cellulose derivative microparticles by first obtaining cellulose microparticles and subjecting the surface of the cellulose microparticles to a chemical treatment to introduce a functional group. As a method for obtaining microparticles using a cellulose derivative as a raw material, Patent Document 5 discloses a method for obtaining cellulose derivative microparticles by dissolving a cellulose derivative in an aqueous solvent, forming a W/O emulsion, and then bringing the emulsion into contact with a poor solvent for the cellulose derivative.

Prior Art Documents

Patent Documents:

Patent Document 1: JP S63-83144A
Patent Document 2: WO 2012/033223
Patent Document 3: WO 2009/123148
Patent Document 4: JP 2003-252903A
Patent Document 5: JP H2-235944A

Summary of the Invention

Problems to be Solved by the Invention

Although Patent Document 1 and Patent Document 2 disclose the methods for producing porous cellulose microparticles, further improved microparticles beyond the above porous cellulose microparticles have been desired as practically available porous microparticles.

In the methods disclosed in Patent Document 3 and Patent Document 4, microparticles the surface of which have been modified into cellulose derivatives can be obtained by introducing functional groups onto the surface of the microparticles, but it is difficult to uniformly introduce the functional groups to the inside of the microparticles. Nonuniform introduction of the functional groups into the microparticles may cause unbalanced physical behavior such as solubility of the microparticles in a solvent, which is not a preferable situation. In addition, according to consideration by the inventors, the cellulose derivative microparticles obtained by the methods disclosed in the patent documents are not porous.

Although microparticles are obtained using a cellulose derivative as a raw material in Patent Document 5, this method is not industrially advantageous for such reasons that available cellulose derivatives are limited to derivatives soluble in aqueous solvents, addition of a thickener is essential in the process of production, and various solvents are used. Furthermore, according to consideration by the inventors, cellulose derivative microparticles obtained by the method disclosed in Patent Document 5 do not have fine pores on the surface of the particles.

Cellulose is a polymer having the characteristic of being chemically stable compared with other synthetic polymers and being less soluble. On the other hand, cellulose derivatives are polymers produced by substituting hydroxy groups of cellulose with other structures and have the characteristic of having solubility in solvents that varies depending on the structures of the substituents and the degree of substitution. In particular, ether cellulose derivatives have unique properties, such as being soluble not only in many organic solvents but also in alcohols and being soluble in water depending on the structure of the substituent, which are not observed for other synthetic polymers, because of etherification of hydroxy groups of cellulose.

Since ether cellulose derivatives have such unique properties, it has been difficult to form the ether cellulose derivatives into microparticles in conventional liquid-liquid emulsions containing equal to or more than two types of solvents without employing combinations of limited solvent species that are poor in versatility and industrial convenience. In particular, it has been extremely difficult to obtain porous microparticles of ether cellulose derivatives having pores both on the surface of and inside the particles.

It is generally desired that microparticles used as carriers or adsorbents have pores both on the surface of and inside the particles and have such pore sizes as to allow the fine pores to incorporate and retain target substances. However, as described above, ether cellulose derivative microparticles obtained using conventional techniques have not satisfied these properties.

An object of the present invention is to provide a practically available porous ether cellulose derivative microparticle having pores both on the surface of and inside the particle.

Another object of the present invention is to provide a composite microparticle including an active ingredient and a porous ether cellulose derivative microparticle having pores both on the surface of and inside the particle.

Means for Solving the Problems

In order to achieve the above objects, the present inventors have reached an invention below after earnest examinations. That is, the present invention has been made in order to achieve at least part of the above objects and can be implemented as the following aspects.

[1] An ether cellulose derivative microparticle has an average particle diameter of 1 to 1,000 $\mu$m, a linseed oil absorption of 50 to 1,000 mL/100 g, and an average surface pore size of 0.05 to 5 $\mu$m.

[2] The ether cellulose derivative microparticle according to [1] may have a pore volume calculated by a mercury intrusion method of 0.05 to 5 cm$^3$/g.

[3] The ether cellulose derivative microparticle according to [1] or [2] may have a sphericity of equal to or more than 80.

[4] The ether cellulose derivative microparticle according to any one of [1] to [3] may have a particle diameter distribution index of 1 to 3.

[5] The ether cellulose derivative microparticle according to any one of [1] to [4] may have a bulk density of 0.05 to 1.0 g/mL.

[6] The ether cellulose derivative microparticle according to any one of [1] to [5] may have a degree of crystallinity of equal to or more than 1%.

[7] In the ether cellulose derivative microparticle according to any one of [1] to [6], an ether cellulose derivative constituting the ether cellulose derivative microparticle may comprise an alkyl cellulose.

[8] In the ether cellulose derivative microparticle according to [7], the alkyl cellulose may comprise ethyl cellulose.

[9] A dispersion comprises the ether cellulose derivative microparticle according to any one of [1] to [8].

[10] A method for producing the ether cellulose derivative microparticle according to any one of [1] to [8] comprises, in a system where phase separation into two phases occurs when an ether cellulose derivative (A), a polymer (B) different from the ether cellulose derivative (A), and an alcohol solvent (C) are mixed together, the two phases comprising a solution phase mainly comprising the ether cellulose derivative (A) and a solution phase mainly comprising the polymer (B), the two separated phases comprising approximately the same solvent, forming an emulsion of the ether cellulose derivative (A), the polymer (B), and the alcohol solvent (C), and bringing the emulsion into contact with a poor solvent (D) for the ether cellulose derivative (A) to precipitate the ether cellulose derivative microparticle.

[11] In the method for producing the ether cellulose derivative microparticle according to [10], the ether cellulose derivative (A) may comprise an ether cellulose derivative having a degree of crystallinity of equal to or more than 2%.

[12] A composite microparticle comprises the ether cellulose derivative microparticle according to any one of [1] to [8] and an active ingredient.

[13] In the composite microparticle according to [12], the active ingredient comprises at least one selected from a physiologically active substance, a perfume, a sweetener, an acidulant, an antioxidant, a preservative, a disinfectant, a coloring agent, an agricultural chemical, a fertilizer, a repellent, an attractant, a fungicide, a sterilant, a germicide, an antimicrobial agent, an antibacterial agent, a preservative agent, an antiseptic agent, a deodorizer, and a lubricant.

[14] The composite microparticle according to [12] or [13] may further comprise on a surface a sustained-release layer configured to suppress release of the active ingredient from the composite microparticle.

[15] A preparation comprises the composite microparticle according to any one of [12] to [14].

Advantageous Effects of Invention

The present invention provides a practically available porous ether cellulose derivative microparticle, which has been hard to obtain by conventional techniques.

The porous ether cellulose derivative microparticle according to an aspect of the present invention has fine pores that open on the surface of the particle and fine pores inside the particle, and the fine pores that open on the surface of the particle has such a surface pore size that allows a target substance to be incorporated from the openings into the particle and retained. Hence, the porous ether cellulose derivative microparticle according to an aspect of the present invention can be suitably used as a carrier that selectively supports a target substance or an adsorbent that selectively adsorbs a target substance in a medium.

In addition, since an ether cellulose derivative constituting the porous ether cellulose derivative microparticle according to an aspect of the present invention has unique dissolution behavior that are not observed for other synthetic polymers, the porous ether cellulose derivative microparticle according to the aspect of the present invention can be used not only as merely a carrier or an adsorbent but also as a concentrating agent in such a way that, for example, the microparticle adsorbs and selectively collects a target component in a medium, and only the microparticle is then selectively dissolved to extract the target component.

A physiologically-inactive active ingredient can be used as the target substance or target component, and the porous ether cellulose derivative microparticle according to an aspect of the present invention can be used as a base material that incorporates the active ingredient into the microparticle and then sustainedly releases the physiologically-inactive active ingredient from the inside to the outside of the microparticle.

Also, since the porous ether cellulose derivative microparticle according to an aspect of the present invention can be used as a base material that incorporates a physiologically-inactive active ingredient into the microparticle and then sustainedly releases the active ingredient from the inside to the outside of the microparticle, a composite microparticle including the porous ether cellulose derivative microparticle according to the aspect of the present invention and a physiologically-inactive active ingredient can have a property of sustainedly releasing the physiologically-inactive active ingredient.

Also, in the present invention, an active ingredient that shows physiological activity, that is, a physiologically active substance, can be used as the target substance or target component. By providing the composite microparticle including the porous ether cellulose derivative microparticle obtained in the present invention and a physiologically active

EP 3 369 749 A1

substance, a composite microparticle for preparation that shows properties of supporting the physiologically active substance and sustainedly releasing the physiologically active substance supported depending on the purpose can be obtained.

Furthermore, since the porous ether cellulose derivative microparticles according to an aspect of the present invention have such a particle diameter that the microparticles are suitably handled practically as a powder, the microparticles can be suitably used as a carrier, an adsorbent, a concentrating agent, or a sustained-release base material of an active ingredient in various applications.

The porous ether cellulose derivative microparticles according to an aspect of the present invention have an extremely high sphericity and a small particle diameter distribution index PDI and thus show good powder flowability. Hence, such microparticles are suitably handled as a powder in applications as various carriers, adsorbents, concentrating agents, and sustained-release base materials of active ingredients. In the case of use as composite microparticles for preparation, composite microparticles having a high eluting ability and sustained-release ability of the physiologically active substance are obtained because of their extremely high sphericity and small particle diameter distribution index PDI.

Brief Description of the Drawings

Fig. 1 is a schematic diagram showing a pore size distribution measured by the mercury intrusion method and showing a method for calculating a pore volume and a mode pore diameter;

Fig. 2 is a diagram showing results of evaluation of sustained-release properties for particles obtained in Example 8 and Comparative Example 3;

Fig. 3 is an image of a porous ethyl cellulose microparticle in Example 6 observed under a scanning electron microscope (FE-SEM);

Fig. 4 is a comparative diagram of water vapor adsorption isotherms of the porous ethyl cellulose microparticles in Example 6, silica gel, and porous calcium silicate, which are porous inorganic particles;

Fig. 5 is a diagram showing load-compression curves when silica gel is compressed obtained through six repeated measurements;

Fig. 6 is a diagram showing load-compression curves when the porous ethyl cellulose microparticles in Example 6 are compressed obtained through six repeated measurements;

Fig. 7 is an FE-SEM image of an ether cellulose derivative microparticle supporting sodium salicylate in Example 9;

Fig. 8 is an FE-SEM image of particles for preparation including a sustained-release layer in Example 19;

Fig. 9 is a diagram showing dissolution rates of sodium salicylate from the particles for preparation including a sustained-release layer in Example 19 in an artificial gastric juice (solution JP1) and an artificial intestinal juice (solution JP2) each containing 0.1% of Tween 80 (registered trademark);

Fig. 10 is a diagram showing dissolution rates of sodium salicylate from a tablet in Example 23 in distilled water containing 0.1% of Tween 80 (registered trademark);

Fig. 11 is a diagram showing dissolution rates of anhydrous caffeine from particles for preparation in Example 16, particles for preparation in Example 20, an orally disintegrating tablet in Example 24, and anhydrous caffeine particles in distilled water containing 0.1% of Tween 80 (registered trademark);

Fig. 12 is a diagram showing dissolution rates of beraprost sodium from mechanical mixtures of particles for preparation and talc in Examples 25 to 27 and mechanical mixtures of crystalline cellulose and talc in Comparative Examples 12 and 13 in distilled water containing 0.1% of Tween 80 (registered trademark);

Fig. 13 is a diagram showing dissolution rates of beraprost sodium from the mechanical mixture of the particles for preparation and talc in Example 25 and mechanical mixtures of particles for preparation and talc for which different time periods of surface modification are employed (Examples 28 to 30) in distilled water containing 0.1% of Tween 80 (registered trademark); and

Fig. 14 is a diagram showing dissolution rates of $\alpha$-tocopherol from particles for preparation in Example 31, a mechanical mixture of particles for preparation and talc in Example 32, particles for preparation in Example 33, and a surface-modified mechanical mixture of particles for preparation and talc (Example 34) in the artificial intestinal juice (solution JP2) containing 1% of Tween 80 (registered trademark).

Mode for Carrying Out the Invention

A porous ether cellulose derivative microparticle (hereinafter also referred to simply as a microparticle) according to an embodiment of the present invention is a porous microparticle made of an ether cellulose derivative. The microparticle is an ether cellulose derivative microparticle that has fine pores on the surface of the particle and inside the particle and has porous appearance.

(Ether Cellulose Derivative)

The ether cellulose derivative in the embodiment of the present invention is a cellulose derivative derived from cellulose on which part of or the whole of hydroxy groups of cellulose is etherified. Specific examples of the ether cellulose derivative made by etherifying part of or the whole of hydroxy groups of cellulose include alkyl celluloses, hydroxyalkyl celluloses, and carboxyalkyl celluloses.

(Alkyl Cellulose)

An alkyl cellulose in the embodiment of the present invention is made by etherifying part of hydroxy groups of cellulose with hydrocarbon groups, such as the ethyl group, the methyl group, and the propyl group. Specific examples of the alkyl cellulose include ethyl cellulose and methyl cellulose.

(Hydroxyalkyl Cellulose)

A hydroxyalkyl cellulose in the embodiment of the present invention is generated by a reaction of hydroxy groups of cellulose with an alkylene oxide, such as ethylene oxide and propylene oxide. Specific examples of the hydroxyalkyl cellulose include hydroxymethyl cellulose, hydroxyethyl cellulose, hydroxypropyl cellulose, hydroxypropyl methyl cellulose, and hydroxyethylmethyl cellulose.

(Carboxyalkyl Cellulose)

A carboxyalkyl cellulose in the embodiment of the present invention is made by etherifying hydroxy groups of cellulose with a carboxyalkyl group, such as the carboxymethyl group, the carboxyethyl group, and the carboxypropyl group. Specific examples of the carboxyalkyl cellulose include carboxymethyl cellulose and carboxyethyl cellulose.

The ether cellulose derivative used in the embodiment of the present invention can be appropriately selected depending on the application of the microparticles, in other words, depending on solubility to be imparted to the resulting microparticles. For example, ethyl cellulose can be selected for an application that necessitates both solubility in alcohols and insolubility in water. Also, hydroxypropyl cellulose can be selected for an application in which a critical temperature such as an LCST (lower critical solution temperature) in solubility in water is to be provided.

In particular, to obtain an ether cellulose derivative microparticle that shows good porosity and superior linseed oil absorption, the ether cellulose derivative is preferably ethyl cellulose, hydroxypropyl cellulose, hydroxypropyl methyl cellulose, carboxymethyl cellulose, or hydroxyethyl cellulose. Ethyl cellulose, hydroxypropyl cellulose, and hydroxypropyl methyl cellulose are more preferable, and ethyl cellulose is particularly preferable.

These ether cellulose derivatives may be used singly or in combination as long as an object of the present invention is achieved.

Although depending on the type (application) of ether cellulose derivative microparticles to be obtained, the ether cellulose derivative in the embodiment of the present invention preferably has a degree of substitution (DS), which represents the average number of etherified hydroxy groups among the hydroxy groups per glucose unit of cellulose, of equal to or more than 0.1. The degree of substitution is more preferably equal to or more than 0.5, even more preferably equal to or more than 1, further preferably equal to or more than 1.5, particularly preferably equal to or more than 2, remarkably preferably equal to or more than 2.2, most preferably equal to or more than 2.45. The upper limit is preferably equal to or less than 2.9, more preferably equal to or less than 2.85, further preferably equal to or less than 2.8. A degree of substitution of less than 0.1 or more than 2.9 is not preferable because handling of the porous ether cellulose derivative microparticles obtained in the embodiment of the present invention becomes difficult in industrial use due to decrease in solubility of the ether cellulose derivative in various solvents and decrease in solvent species that can dissolve the ether cellulose derivative. Cellulose has three hydroxy groups per glucose unit, and the maximum value of the degree of substitution is three.

The ether cellulose derivative in the embodiment of the present invention preferably includes the ethoxy group. In the case where the ethoxy group is included, the degree of substitution with the ethoxy group is more preferably equal to or more than 2, further preferably equal to or more than 2.2, particularly preferably equal to or more than 2.45. The degree of substitution with the ethoxy group is preferably equal to or less than 2.8. If the degree of substitution with the ethoxy group is within the above range, solubility of the ether cellulose derivative in various solvents is improved, and microparticles usable in wide applications can be obtained.

Furthermore, a viscosity of a solution obtained by dissolving 5 parts by mass of the ether cellulose derivative in the embodiment of the present invention in a mixed solvent of 80 parts by mass of toluene and 20 parts by mass of ethanol under a temperature condition of 25°C is preferably as follows. That is, the lower limit is preferably equal to or more than 1 m Pa·s, more preferably equal to or more than 3 m Pa·s, further preferably equal to or more than 5 m Pa·s, particularly preferably equal to or more than 8 m Pa·s, remarkably preferably equal to or more than 12 m Pa·s, most preferably equal to or more than 20 m Pa·s. The upper limit is preferably equal to or less than 500 m Pa·s, more preferably equal to or less than 350 m Pa·s, further preferably equal to or less than 250 m Pa·s, particularly preferably equal to or less than 110 m Pa·s, remarkably preferably equal to or less than 70 m Pa·s, most preferably equal to or less than 55 m Pa·s.

It is preferable that the ether cellulose derivative having the solution viscosity within the above range constitute the microparticles because, in an application in which the porous ether cellulose derivative microparticles according to the embodiment of the present invention is partially or entirely subjected to a dissolution treatment, the degree of the treatment is easily adjusted depending on the purpose, which improves usefulness of the porous ether cellulose derivative microparticles according to the embodiment of the present invention. For example, in the case of use as surface-modified particles for preparation described later, particles for preparation having a reasonably modified form of the surface of the microparticles are easily obtained.

The microparticles according to the embodiment of the present invention may contain components other than the ether cellulose derivative as long as the objects of the present invention are not impaired. In this case, the content by percentage of the ether cellulose derivative is preferably equal to or more than 50 mass%, more preferably equal to or more than 70 mass%, further preferably equal to or more than 80 mass%, particularly preferably equal to or more than 90 mass%, remarkably preferably equal to or more than 95 mass%. It is preferable that approximately no component other than the ether cellulose derivative be contained, and the upper limit of the content of the ether cellulose derivative is 100 mass%.

The porous ether cellulose derivative microparticle obtained in the embodiment of the present invention has many pores both on the surface of and inside the particle.

In the embodiment of the present invention, the statement that the microparticle has pores both on the surface of and inside the particle means that the microparticle has a characteristic of having fine pores (surface pores) that open on the surface of the particle and having voids (inner pores) inside the particle.

Each of the surface pores and the inner pores of the porous ether cellulose derivative microparticle obtained in the embodiment of the present invention may be isolated from one another, or such a porous structure may be formed that some of the pores continuously communicate with one another. The porous structure in which the pores continuously communicate with one another is preferable because, such a structure increases linseed oil absorption described later and increases the amount of a target component that can be incorporated into the particle.

Furthermore, the porous ether cellulose derivative microparticle obtained in the embodiment of the present invention has a characteristic of having an average surface pore size of 0.05 to 5 $\mu$m.

The average surface pore size here means the average size of openings (surface openings) of the above surface pores on the surface of the microparticle. The lower limit of the average surface pore size is preferably equal to or more than 0.07 $\mu$m, more preferably equal to or more than 0.1 $\mu$m, further preferably equal to or more than 0.3 $\mu$m, particularly preferably equal to or more than 0.5 $\mu$m, remarkably preferably equal to or more than 0.7 $\mu$m. The upper limit is preferably equal to or less than 4 $\mu$m, more preferably equal to or less than 3 $\mu$m, further preferably equal to or less than 2.5 $\mu$m, particularly preferably equal to or less than 2 $\mu$m, remarkably preferably equal to or less than 1.5 $\mu$m.

If the average surface pore size takes on a too small value, such as a value less than 0.05 $\mu$m, it is difficult for components having high molecular weights to enter the pores and to be incorporated into the particle and retained. On the other hand, if the average surface pore size takes on a too large value, such as a value larger than 5 $\mu$m, capillary force of the fine pores is abated, which hinders the action of incorporating a target component into the particle. Such a situation is not preferable in view of practical use of the porous ether cellulose derivative microparticle according to the embodiment of the present invention.

The above average surface pore size is the average value of surface pore sizes measured using images observed under a scanning electron microscope (FE-SEM). Specifically, the surface of a particle is magnified at a high magnification and observed under such a magnification and field that equal to or more than 10 surface pores are present in an FE-SEM image, and the diameters (pore sizes) of a total of 100 surface pores on the surfaces of equal to or more than 2 microparticles are measured. The average surface pore size is determined as the arithmetic mean from the equation below. Although depending on the surface pore size, such a magnification of the FE-SEM can be within the range of 5,000 to 100,000 times. Specific examples thereof include equal to or more than 100,000 times in the case where the surface pore size is in the range of 0.05 to less than 0.1 $\mu$m, equal to or more than 50,000 times in the case of 0.1 to less than 0.3 $\mu$m, equal to or more than 30,000 times in the case of 0.3 to less than 0.5 $\mu$m, equal to or more than 10,000 times in the case of 0.5 to less than 1.0 $\mu$m, and equal to or more than 5,000 times in the case of 1.0 to 5 $\mu$m. In the case where a surface pore is not perfect circular (for example, elliptic) in an image, its maximum diameter is measured as the pore size. In the case where fine pores communicate with one another to form an irregular shape, the maximum diameter of the connected pore is measured as the pore size.

[Math. 1]

[Math.1]

$$P = \left(\sum_{i=1}^{n} P_i\right)/n$$

(In the equation, Pi represents the pore size of an individual surface pore, P represents the average surface pore size, and n represents the number of measurements, which is 100 in the present embodiment.)

The porous ether cellulose derivative microparticle according to the embodiment of the present invention is characterized by the fact that the microparticle has not only the above surface pores but also the inner pores inside the particle. Hence, the microparticle according to the embodiment of the present invention is deemed to be able to incorporate a target substance into the microparticle through the surface openings and retain the substance.

The degree of incorporation and retention of a target substance by the porous ether cellulose derivative microparticle according to the embodiment of the present invention is deemed to be affected by the porosity of the microparticle. However, it is difficult to directly measure the porosity of the microparticle. Hence, indices such as adsorption of a gas per unit weight by the BET method and linseed oil absorption, which is a test method for pigments defined by Japan Industrial Standard (refined linseed oil method: Japan Industrial Standard (JIS) K 5101-13-1), are used as indirect indices to the porosity.

Since the specific surface area method by the BET method strongly depends on the average particle diameter, the linseed oil absorption is used as the index to the porosity of the microparticle in the embodiment of the present invention. The linseed oil absorption is used as the index to the porosity of the microparticle according to the embodiment of the present invention also in view of the fact that the linseed oil absorption well reflects properties such as support, adsorption, and concentration of the porous ether cellulose derivative microparticle according to the embodiment of the present invention.

The value of the linseed oil absorption is measured in accordance with Japan Industrial Standard (JIS) K 5101-13-1. Specifically, porous microparticles are placed on a glass plate, refined linseed oil is slowly added from a burette, and the refined linseed oil is kneaded into the porous microparticles with a palette knife each time. This procedure is repeated to completely mix the refined linseed oil with the porous microparticles, and the volume of the linseed oil consumed is read on the burette at the end point where the paste has got a smooth hardness. The resulting paste can be spread without cracking or crumbling and slightly adheres to the glass plate. The linseed oil absorption is represented as the volume of the linseed oil consumed per 100 g of the porous microparticles.

The porous ether cellulose derivative microparticles according to the embodiment of the present invention have a characteristic of having a linseed oil absorption of 50 to 1,000 mL/100 g. Its lower limit is preferably equal to or more than 75 mL/100 g, more preferably equal to or more than 100 mL/100 g, further preferably equal to or more than 150 mL/100 g, particularly preferably equal to or more than 200 mL/100 g, remarkably preferably equal to or more than 250 mL/100 g. Its upper limit is preferably equal to or less than 900 mL/100 g, more preferably equal to or less than 800 mL/100 g, further preferably equal to or less than 700 mL/100 g, particularly preferably equal to or less than 600 mL/100 g, remarkably preferably equal to or less than 500 mL/100 g.

It is not practically preferable that the linseed oil absorption take on a too small value, such as a value less than 50 mL/100 g, because the amount of a target substance that can be incorporated into the microparticles decreases, which increases the amount of the microparticles required in, for example, an application in which the microparticles are used as an adsorbent. On the other hand, it is not preferable in view of an environment for handling the microparticles that the linseed oil absorption is too large because the bulk density of such microparticles tends to be small, resulting in extremely easy scattering of the microparticles.

The porous ether cellulose derivative microparticles according to the embodiment of the present invention preferably have a pore volume calculated by the mercury intrusion method in the range of 0.05 to 5 $cm^3$/g. Its lower limit is preferably equal to or more than 0.08 $cm^3$/g, more preferably equal to or more than 0.1 $cm^3$/g, further preferably equal to or more than 0.25 $cm^3$/g, particularly preferably equal to or more than 0.4 $cm^3$/g, remarkably preferably equal to or more than 0.5 $cm^3$/g. Its upper limit is preferably equal to or less than 4 $cm^3$/g, more preferably equal to or less than 3.5 $cm^3$/g, further preferably equal to or less than 3 $cm^3$/g, particularly preferably equal to or less than 2.5 $cm^3$/g, remarkably preferably equal to or less than 2 $cm^3$/g.

It is not preferable that the pore volume be less than 0.05 $cm^3$/g because the small pore volume results in a small volume of a target substance to be retained. Also, it is not preferable that the pore volume exceed 5 $cm^3$/g because it becomes difficult to control release behavior of a retained substance and thus to obtain homogeneous sustained-release ability.

In the embodiment of the present invention, the pore volume calculated by the mercury intrusion method means an integrated value of the volume derived from the fine pores excluding the volume derived from gaps between particles. That is, on an integrated pore size distribution curve with the horizontal axis indicating the pore diameter R and the vertical axis indicating the integrated pore volume Vp, the integrated value excluding the volume appearing in a region near the average particle diameter or equal to or larger than the average particle diameter is meant.

In the embodiment of the present invention, the mode pore diameter calculated by the mercury intrusion method is preferably 0.2 to 2.5 times as large as the average surface pore size. Its lower limit is preferably equal to or more than 0.3 times, more preferably equal to or more than 0.5 times, further preferably equal to or more than 0.6 times, particularly preferably equal to or more than 0.7 times, remarkably preferably equal to or more than 0.8 times. Its upper limit is preferably equal to or less than 2.2 times, more preferably equal to or less than twice, further preferably equal to or less than 1.7 times, particularly preferably equal to or less than 1.5 times, remarkably preferably equal to or less than 1.3 times.

It is not preferable that the mode pore diameter be less than 0.2 times as large as the average surface pore size because the pore diameter inside a particle is small compared with the surface openings, which reduces retention efficiency of a target substance inside the particle. Also, it is not preferable that the mode pore diameter be larger than 2.5 times as large as the average surface pore size because the action of retaining a substance inside the particle by capillary force tends to be weak, which makes it difficult to control the release behavior of a target substance.

In the embodiment of the present invention, the mode pore diameter calculated by the mercury intrusion method is a pore size indicated by the peak top of the highest peak among peaks derived from the fine pores on a differential pore size distribution curve measured by the mercury intrusion method with the horizontal axis indicating the pore diameter R and the vertical axis indicating the differential pore volume dVp/d(log R). That is, the pore size indicated by the peak top of the highest peak among peaks appearing not near the average particle diameter but near the average surface pore size in the pore size distribution is meant.

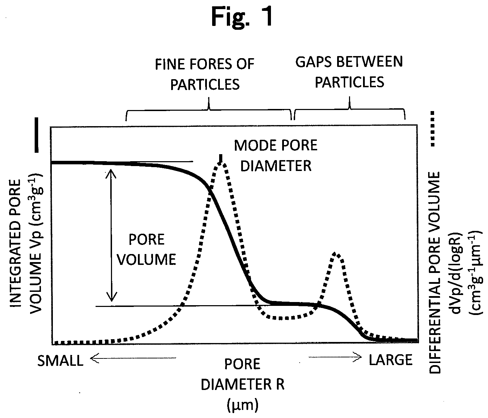

Fig. 1 is a schematic diagram showing the pore size distribution measured by the mercury intrusion method and showing a method for calculating the pore volume and the mode pore diameter in the embodiment of the present invention.

In addition, the openings on the surface of the porous ether cellulose derivative microparticles according to the embodiment of the present invention are preferably formed densely, so that the particles function as a carrier or an adsorbent by incorporating another component through the openings on their surface.

Specifically, the average inter-surface-pore distance, which represents the distance between adjacent openings on the surface of the particles, is preferably 0.05 to 2.5 times as large as the average surface pore size. Its lower limit is preferably equal to or more than 0.07 times, more preferably equal to or more than 0.1 times, further preferably equal to or more than 0.2 times, particularly preferably equal to or more than 0.3 times, remarkably preferably equal to or more than 0.4 times. Its upper limit is preferably equal to or less than 2.3 times, more preferably equal to or less than twice, further preferably equal to or less than 1.8 times, particularly preferably equal to or less than 1.5 times, remarkably preferably equal to or less than 1.3 times.

It is not practically preferable that the surface pores be formed too densely, that is, the average inter-surface-pore distance take on a too small value, such as a value less than 0.05 times as large as the average surface pore size, because the resulting microparticles become fragile. On the other hand, it is not preferable that the average inter-surface-pore distance exceed 2.5 times of the above standard because the number of openings on the surface of the particles becomes small, which reduces efficiency in incorporation of an active ingredient into the microparticles.

The average inter-surface-pore distance can be determined from the FE-SEM images used for measuring the average surface pore size. The inter-surface-pore distance here means the shortest distance to the closest fine pore among distances between an arbitrary surface pore and adjacent fine pores. The average inter-surface-pore distance is the arithmetic mean determined from the equation below through measuring the distance from each of 30 surface pores randomly selected to an adjacent fine pore closest to the fine pore in the above images.

[Math. 2]

$$[\text{Math.2}]$$

$$L = \left( \sum_{i=1}^{n} L_i \right) / n$$

(In the equation, $L_i$ represents the shortest distance between an arbitrary fine pore and the closest adjacent fine pore, L represents the average inter-surface-pore distance, and n represents the number of measurements, which is 30 in the present embodiment.)

The porous ether cellulose derivative microparticles obtained in the embodiment of the present invention have a characteristic of having the fine pore structure as described above and having an arithmetic mean (average particle diameter) of particle diameters of 1 to 1,000 $\mu$m measured using the images of the particles observed under a scanning electron microscope. Its lower limit is preferably equal to or more than 10 $\mu$m, more preferably equal to or more than 25 $\mu$m, further preferably equal to or more than 50 $\mu$m, particularly preferably equal to or more than 60 $\mu$m, remarkably preferably equal to or more than 70 $\mu$m. Its upper limit is preferably equal to or less than 800 $\mu$m, more preferably equal to or less than 600 $\mu$m, further preferably equal to or less than 500 $\mu$m, particularly preferably equal to or less than 400 $\mu$m, remarkably preferably equal to or less than 300 $\mu$m.

It is not preferable that the average particle diameter be smaller than 1 $\mu$m because the resulting porous ether cellulose derivative microparticles easily coagulate or get lumpy, which impairs flowing characteristics of the microparticles. On the other hand, it is not practically preferable that the average particle diameter exceed 1,000 $\mu$m because the resulting porous ether cellulose derivative microparticles easily settle.

The above average particle diameter is the average value of particle diameters measured using images observed under an FE-SEM in substantially the same manner as for the surface pore size. Specifically, observations are made under such a magnification and field that 2 to less than 100 microparticles are present in an FE-SEM image, and the diameters (particle diameters) of 100 microparticles in a plurality of fields are measured. The average particle diameter is determined as the arithmetic mean from the equation below. Although depending on the particle diameters of the microparticles, such a magnification of the FE-SEM can be within the range of 50 to 5,000 times. Specific examples thereof include equal to or more than 1,000 times in the case where the particle diameter is in the range of 1 to less than 20 $\mu$m, equal to or more than 500 times in the case of 20 to less than 50 $\mu$m, equal to or more than 300 times in the case of 50 to less than 75 $\mu$m, equal to or more than 150 times in the case of 75 to less than 200 $\mu$m, equal to or more than 100 times in the case of 200 to less than 500 um, and equal to or more than 50 times in the case of 500 to 1,000 $\mu$m. In the case where a microparticle is not perfect circular (for example, elliptic) in an image, its maximum diameter is measured as the particle diameter. In the case where microparticles gather to form an irregular aggregate, the diameter of a microparticle serving as the minimum unit constituting the aggregate is measured as the particle diameter. In the case where the aggregate is formed by fusion of a plurality of microparticles and shows no certain boundaries between the microparticles, the maximum diameter of the fused body is measured as the particle diameter.

[Math. 3]

$$[\text{Math.3}]$$

$$D = \left( \sum_{i=1}^{n} D_i \right) / n$$

(In the equation, Di represents the particle diameter of an individual microparticle, D represents the average particle diameter, and n represents the number of measurements, which is 100 in the present embodiment.)

The sphericity of the porous ether cellulose derivative microparticles according to the embodiment of the present invention is preferably equal to or more than 80, more preferably equal to or more than 85, further preferably equal to or more than 90, particularly preferably equal to or more than 95, remarkably preferably equal to or more than 98. If the sphericity is low, the flowing characteristics of the resulting microparticles tend to be impaired. Such microparticles are poor in handleability and are thus not preferable.

The sphericity of the porous ether cellulose derivative microparticles according to the embodiment of the present invention is the arithmetic mean of the sphericities of 30 microparticles randomly selected in images observed under an FE-SEM and can be calculated according to the equation below. The sphericity of an individual microparticle is the ratio between the major axis (maximum diameter) and the minor axis that intersects perpendicularly to the major axis at the center of the major axis of the individual microparticle. The sphericity can be calculated according to the equation below. The major axis and the minor axis of the individual microparticle can be measured under the above magnification and field for measuring the particle diameters to determine the average particle diameter.

[Math. 4]

[Math.4]

$$S_m = \left(\sum_{i=1}^{n} S_i\right)/n$$

$$S_i = (a_i \, / \, b_i) * 100$$

(In the equation, Sm represents the average sphericity (%), Si represents the sphericity of an individual microparticle, ai represents the minor axis of the individual microparticle, bi represents the major axis of the individual microparticle, and n represents the number of measurements, which is 30 in the present embodiment.)

Also, the porous ether cellulose derivative microparticles according to the embodiment of the present invention have a particle diameter distribution index (PDI), which is an index to the breadth of the particle diameter distribution, of preferably 1 to 3, more preferably 1 to 2.5, further preferably 1 to 2.0, particularly preferably 1 to 1.8, remarkably preferably 1 to 1.5. The theoretical lower limit value of the PDI is 1. A small PDI leads to small apparent differences in volumes between microparticles and small unevenness of the ability to retain a target component, and the support, adsorption, and concentration performance of the microparticles become more reliable. Microparticles having a large width of the particle diameter distribution and a large PDI are not preferable not only because the retention ability varies greatly but also because microparticles having large particle diameters and small particle diameters are separated when the microparticles are handled as a powder.

The PDI of the porous ether cellulose derivative microparticles according to the embodiment of the present invention is calculated from the equation below using the results of the measurements of the particle diameters performed when calculating the average particle diameter.

[Math. 5]

[Math.5]

$$PDI = \frac{\left(\sum_{i=1}^{n} D_i^{\,4}\right)/\left(\sum_{i=1}^{n} D_i^{\,3}\right)}{\left(\sum_{i=1}^{n} D_i\right)/n}$$

(In the equation, Di represents the particle diameter of an individual particle, PDI represents the particle diameter distribution index, and n represents the number of measurements, which is 100 in the present embodiment.)

Also, the porous ether cellulose derivative microparticles according to the embodiment of the present invention have a bulk density of preferably equal to or more than 0.05 g/mL, more preferably equal to or more than 0.08 g/mL, further preferably equal to or more than 0.09 g/mL, particularly preferably equal to or more than 0.10 g/mL. Its upper limit is preferably equal to or less than 1.0 g/mL, more preferably equal to or less than 0.8 g/mL, further preferably equal to or less than 0.6 g/mL, particularly preferably equal to or less than 0.5 g/mL, remarkably preferably equal to or less than 0.45 g/mL.

It is not preferable that the bulk density take on a small value, such as a value smaller than 0.05 g/mL, because the microparticles are easily scattered and may deteriorate the working environment. On the other hand, it is not preferable that the bulk density take on a large value, such as a value larger than 1.0 g/mL, because the microparticles easily settle and are separated in a medium.

The bulk density of the porous ether cellulose derivative microparticles according to the embodiment of the present invention can be calculated according to the equation below through precisely weighing 4.00 g of the microparticles (the mass of the microparticles at this time is represented as W), then gently putting the weighed microparticles having a mass of W (g) into a 100-mL graduated cylinder (with a minimum scale of 1 mL), and visually measuring the volume V (mL) of the powder layer. At this time, a similar measurement operation is performed again using a 50-mL graduated cylinder (with a minimum scale of 1 mL) in the case where the volume of the powder layer is 25 to less than 50 mL, a 25-mL graduated cylinder (with a minimum scale of 0.5 mL) in the case where the volume of the powder layer is 10 to less than 25 mL, a 10-mL graduated cylinder (with a minimum scale of 0.2 mL) in the case of 5 to less than 10 mL, or a 5-mL graduated cylinder (with a minimum scale of 0.1 mL) in the case of less than 5 mL, and the bulk density of the microparticles is calculated using the reading obtained by the re-measurement as the volume of the powder layer.

[Math. 6]

$$[\text{Math.6}]$$

$$\text{Bulk density} = \frac{W}{V}$$

(In the equation, W represents the mass of the microparticles, and V represents the volume of the powder layer measured with a graduated cylinder.)

The porous ether cellulose derivative microparticles according to the embodiment of the present invention preferably have a degree of crystallinity of the microparticles of equal to or more than 1%. Its lower limit is more preferably equal to or more than 1.5%, further preferably equal to or more than 2%, particularly preferably equal to or more than 2.5%, remarkably preferably equal to or more than 3%, most preferably equal to or more than 3.5%. The porous ether cellulose derivative microparticles showing such a degree of crystallinity seem to have a porous structure including particularly developed surface pores and inner pores of the microparticles and are suitable for an application in which a target substance is incorporated from the openings on the surface of the microparticles into the particles and retained. The upper limit of the degree of crystallinity of the microparticles can be selected from various values depending on the application, and is preferably equal to or less than 60%, more preferably equal to or less than 40%, further preferably equal to or less than 20%, particularly preferably equal to or less than 15%, remarkably preferably equal to or less than 10%, most preferably equal to or less than 5%. Making the degree of crystallinity of the microparticles be within the above range improves solubility of the microparticles in various solvents and allows the microparticles to show solubility in a wide range of solvents, so that the range of the applications of the microparticles according to the embodiment of the present invention is broadened. For example, the microparticles can be suitably used in an application in which the microparticles are used as a concentrating agent such that a target component in a medium is adsorbed by the microparticles and selectively collected, and the target component is extracted by selectively dissolving only the microparticles.

In the embodiment of the present invention, the degree of crystallinity of the porous ether cellulose derivative microparticles can be determined as described below. That is, a measurement is performed by X-ray powder diffraction using CuK$\alpha$ as the X-ray at diffraction angles of the X-ray of 5 to 70° and a scan rate of 2°/min with a sampling width of 0.02°, and the diffraction pattern is subjected to waveform separation into the crystalline portion and the amorphous portion. The degree of crystallinity is then calculated by (the scattering intensity from the crystalline portion)/(the total scattering intensity) $\times$ 100.

The porous ether cellulose derivative microparticles according to the embodiment of the present invention may be used as a powder or a dispersion formed by dispersing the microparticles in a desired dispersion medium.

The dispersion medium of the dispersion is not particularly limited as long as the ether cellulose derivative is not completely dissolved or loses its form as microparticles. Examples of the dispersion medium include water, alcohol solvents, polyhydric alcohol solvents, ketone solvents, ether solvents, aliphatic hydrocarbons, and alicyclic hydrocarbons. Specific examples of these solvents include water, ethanol, methanol, 1-propanol, 2-propanol, ethylene glycol, diethylene glycol, glycerin, acetone, methyl ethyl ketone, ethyl ether, tetrahydrofuran, pentane, hexane, heptane, octane, nonane, n-decane, n-dodecane, cyclohexane, cyclohexene, methylcyclohexane, and petroleum naphtha.

The porous ether cellulose derivative microparticles may be produced by a conventionally known technique as long as the porous ether cellulose derivative microparticles according to the embodiment of the present invention are obtained. Examples of the method include a method of obtaining microparticles by producing an O/W emulsion or a W/O/W emulsion of an ether cellulose derivative and then distilling off the solvent or diffusion-removing the organic solvent from the oil phase using the osmotic pressure; a method of reducing an ether cellulose derivative resin to a powder by a mechanical powdering treatment such as ball milling, bead milling, jet milling, and powdering with a mortar; a forced melt-kneading method of melt-kneading an ether cellulose derivative resin and a sea-component resin and removing only the sea-component resin; a method of dissolving an ether cellulose derivative in a highly volatile solvent and forming the solution into microparticles by spray drying or electrostatic spraying; a method of dissolving an ether cellulose derivative in a solvent and cooling the solution to cause precipitation; and a method of forming a porous structure using a foaming agent when forming microparticles by any of the above methods.

Among these methods, the following method for forming microparticles using the phase separation phenomenon of a polymer solution is most preferable. Employing the method for forming microparticles using the phase separation phenomenon of a polymer solution is preferable in that porous ether cellulose derivative microparticles having fine openings on the surface of the particles and fine pores inside the particles formed by spontaneous self-organization of the ether cellulose derivative are obtained.

The method for forming microparticles using the phase separation phenomenon of a polymer solution includes, in a system where phase separation into two phases occurs when an ether cellulose derivative (A), a polymer (B) different from the ether cellulose derivative, and an alcohol solvent (C) are mixed together, the two phases including a solution phase mainly containing the ether cellulose derivative (A) and a solution phase mainly containing the polymer (B), the two separated phases containing approximately the same solvent, forming an emulsion of the ether cellulose derivative (A), the polymer (B), and the alcohol solvent (C), and bringing the emulsion into contact with a poor solvent (D) for the ether cellulose derivative (A) to precipitate ether cellulose derivative microparticles.

Since ether cellulose derivatives have unique dissolution properties, it has been difficult in conventional methods employing liquid-liquid emulsions each containing equal to or more than two types of solvents to create such conditions as to prevent the ether cellulose derivative from being dissolved in one solvent using a combination of general-purpose solvents that can exhibit liquid-liquid phase separation. Hence, it has been extremely difficult to obtain porous ether cellulose derivative microparticles having surface pores that open on the surface of the microparticles and having inner pores.

The inventors of the present application have found that a system exists where, when the ether cellulose derivative (A) and the polymer (B) different from the ether cellulose derivative are mixed and dissolved in the alcohol solvent (C), separation into two phases of the solution phase mainly containing the ether cellulose derivative (A) and the solution phase mainly containing the polymer (B) occurs with the two separated phases containing approximately the same solvent. Furthermore, surprisingly, it has been found that emulsifying the system having been separated into two phases and then bringing the emulsion into contact with the poor solvent (D) that is soluble in the alcohol solvent (C) and serves as a poor solvent for the ether cellulose derivative (A) provides porous ether cellulose derivative microparticles having surface pores that open on the surface and inner pores. The statement that the two separated phases contain approximately the same solvent includes not only the case where the solvent of both phases is the alcohol solvent (C) but also the case where another component is mixed in the alcohol solvent (C).

When the emulsion is brought into contact with the poor solvent (D), the ether cellulose derivative is deemed to form a porous structure by spontaneous self-organization in the process of extracting the alcohol solvent (C) from the droplet phase mainly containing the ether cellulose derivative (A) to solidify the ether cellulose derivative (A) in a granular form.

Examples of the polymer (B) include thermoplastic resins and thermosetting resins among polymers different from the ether cellulose derivative (A), and thermoplastic resins are preferable in that these resins are soluble in the alcohol solvent (C). Specific examples thereof include polyvinylpyrrolidone, polyethylene oxide, polyethylene glycol, and poly(vinyl alcohol) (completely saponified or partially saponified poly(vinyl alcohol) may be used). Polyvinylpyrrolidone or poly(vinyl alcohol) (completely saponified or partially saponified poly(vinyl alcohol) may be used) can be preferably used because the particle diameter of the resulting porous ether cellulose derivative microparticles is easily controlled and because their sphericity is enhanced.

The weight-average molecular weight of the polymer (B) is preferably equal to or more than 1,000. Use of such a polymer (B) is deemed to induce phase separation into two phases of the ether cellulose derivative (A) solution phase and the polymer (B) solution phase. Also, use of such a polymer (B) is deemed to provide when the emulsion is formed the porous ether cellulose derivative microparticles having surface pores and inner pores and having a high sphericity.

The weight-average molecular weight of the polymer (B) is preferably in the range of 1,000 to 10,000,000. A more preferable upper limit is equal to or less than 5,000,000, further preferably equal to or less than 2,000,000, particularly preferably equal to or less than 1,000,000. In view of ease of phase separation, a more preferable lower limit is equal to or more than 2,000, further preferably equal to or more than 5,000, particularly preferably equal to or more than 10,000, remarkably preferably equal to or more than 20,000.

The weight-average molecular weight here means a weight-average molecular weight in terms of polyethylene glycol measured by gel permeation chromatography (GPC) employing water as the solvent. Dimethylformamide is used as the solvent in the case where the measurement cannot be carried out using water as the solvent, tetrahydrofuran is used in the case where the measurement still cannot be carried out, and hexafluoroisopropanol is used in the case where the measurement still cannot be carried out.

The alcohol solvent (C) is a solvent that dissolves the ether cellulose derivative (A) and the polymer (B). The solvent that dissolves the polymers here means that the ether cellulose derivative (A) and the polymer (B) are dissolved in the alcohol solvent (C) at a concentration of more than 1 mass% at a temperature at which the operation is actually performed, that is, at which the ether cellulose derivative (A) and the polymer (B) are mixed and dissolved.

The alcohol solvent (C) can preferably dissolve the ether cellulose derivative (A) at a temperature in the step of bringing the emulsion into contact with the poor solvent (D) to provide the microparticles of the ether cellulose derivative (A).

Preferable examples of the alcohol solvent (C) include ethanol, methanol, 2-propanol, and 1-propanol. A plurality of types of these solvents may be used, or these solvents may be used singly. Ethanol is more preferable in view of solubility of the ether cellulose derivative (A) and safety in the working environment.

The poor solvent (D) is a poor solvent for the ether cellulose derivative (A), and the solubility of the ether cellulose derivative (A) in the poor solvent is equal to or less than 1 mass% at a temperature at which the poor solvent is brought into contact. The solubility in the poor solvent (D) is more preferably equal to or less than 0.5 mass%, further preferably equal to or less than 0.1 mass%.

Some ether cellulose derivatives (A) show LCSTs in combination with the solvent. Hence, for a solvent that shows an LCST in combination with the ether cellulose derivative (A) to be used, it is determined at a temperature equal to or higher than the LCST whether the solvent functions as a poor solvent.

In addition, the poor solvent (D) is preferably a solvent that dissolves the polymer (B). With this constitution, the ether cellulose derivative (A) can be efficiently precipitated as microparticles. Also, the poor solvent (D) is preferably a solvent that is soluble in the alcohol solvent (C) and can be uniformly mixed with the alcohol solvent (C) at an arbitrary ratio.

Although depending on the types of the ether cellulose derivative (A), the polymer (B), and the alcohol solvent (C), specific examples of the poor solvent (D) include at least one selected from water; polyhydric alcohol solvents such as ethylene glycol and diethylene glycol; aliphatic hydrocarbon solvents such as pentane, hexane, heptane, octane, nonane, n-decane, n-dodecane, cyclohexane, and cyclopentane; and aromatic hydrocarbon solvents such as benzene and xylenes. Water, ethylene glycol, or diethylene glycol is preferable because the ether cellulose derivative (A) is efficiently formed into microparticles, and water is most preferable because water is superior in view of safety in the working environment.

To create conditions under which phase separation easily occurs, the SP values (solubility parameters) of the ether cellulose derivative (A) and the polymer (B) preferably differ widely. In this case, the difference in the SP values is equal to or more than $1 \ (J/cm^3)^{1/2}$, more preferably equal to or more than $2 \ (J/cm^3)^{1/2}$, further preferably equal to or more than $3 \ (J/cm^3)^{1/2}$, particularly preferably equal to or more than $5 \ (J/cm^3)^{1/2}$, remarkably preferably equal to or more than $8 \ (J/cm^3)^{1/2}$. If the difference in the SP values is within this range, phase separation easily occurs. Also, easy phase separation enables microparticles having a higher content by percentage of the ether cellulose derivative (A) to be obtained. Although the difference is not particularly limited as long as both of the ether cellulose derivative (A) and the polymer (B) are soluble in the alcohol solvent (C), the upper limit of the difference in the SP values is preferably equal to or less than $20 \ (J/cm^3)^{1/2}$, more preferably equal to or less than $15 \ (J/cm^3)^{1/2}$, further preferably equal to or less than $10 (J/cm^3)^{1/2}$. It is not preferable that the difference in the SP values be larger than this range because either of the ether cellulose derivative (A) and the polymer (B) may not be soluble in the alcohol solvent (C).

The SP values here are calculated on the basis of the Fedors' estimation method based on the cohesive energy density and the molar volume (hereinafter also referred to as the calculation method) (Yamamoto, Hideki. SP-chi Kiso·Ouyou To Keisanhouhou, Johokiko Co., Ltd., March 31, 2005). In the case where calculation by this method is not possible, SP values calculated by an experimental method based on judgment of whether a solubility parameter is soluble in a known solvent (hereinafter also referred to as the experimental method) are used instead (Brand, J. Polymer Handbook, fourth edition, Wiley, 1998).

To select conditions under which phase separation is achieved, whether phase separation occurs is distinguished on the basis of a three-component phase diagram that can be made through a simple preparatory test to observe the states created by varying the ratio among the three components including the ether cellulose derivative (A), the polymer (B), and the alcohol solvent (C) that dissolves these two components.

To make the three-component phase diagram, the ether cellulose derivative (A), the polymer (B), and the alcohol solvent (C) are mixed and dissolved at an arbitrary ratio and allowed to stand still to judge whether an interface is formed. This operation is carried out at least at three ratios, preferably equal to or more than five ratios, more preferably equal to or more than ten ratios. By determining the region where two-phase separation occurs and the region where only one phase forms, the conditions under which phase separation occurs can be found.

At this time, to judge whether phase separation has occurred, the ratio among the ether cellulose derivative (A), the polymer (B), and the alcohol solvent (C) is adjusted to an arbitrary ratio at the temperature and pressure at which the emulsion is actually formed, the ether cellulose derivative (A) and the polymer (B) are completely dissolved, the solution is stirred sufficiently and allowed to stand still for three days, and whether macroscopic phase separation occurs is determined. However, in the case where a sufficiently stable emulsion is formed, macroscopic phase separation may not occur even after being left standing for three days. In this case, the occurrence of phase separation is distinguished on the basis of microscopic phase separation observed under an optical microscope or a phase-contrast microscope.

The lower limit of the concentration of each of the ether cellulose derivative (A) and the polymer (B) in the alcohol solvent (C) is preferably more than 1 mass%, more preferably equal to or more than 2 mass%, further preferably equal to or more than 3 mass%, more preferably equal to or more than 5 mass% relative to the total mass, inevitably to the extent that the ether cellulose derivative (A) and the polymer (B) can be dissolved in the alcohol solvent (C). The upper limit of each concentration is preferably equal to or less than 50 mass%, more preferably equal to or less than 30 mass%, further preferably equal to or less than 20 mass%.

The interfacial tension between the two phases of the ether cellulose derivative (A) solution phase and the polymer (B) solution phase obtained as a result of phase separation is small because both phases are mainly made of the alcohol solvent (C). The emulsion to be formed is stable and has a very narrow droplet size distribution because of this property, resulting in a high sphericity and a narrow particle diameter distribution of the resulting porous ether cellulose derivative microparticles. This tendency is notable when both of the ether cellulose derivative (A) and the polymer (B) are dissolved in a single solvent serving as the alcohol solvent (C) to cause phase separation.

The interfacial tension between the two phases formed by phase separation is too small to directly measure by the common hanging-drop method, in which another type of solution is added to a solution to carry out measurements, but the interfacial tension can be estimated from the surface tension of each phase in the air. Letting the surface tensions of the respective phases in the air be $r_1$ and $r_2$, the interfacial tension $n_{1/2}$ can be estimated as the absolute value $r_{1/2} = r_1 - r_2$.

In this case, a preferable range of $r_{1/2}$ is represented by an upper limit of equal to or less than 10 mN/m, more preferably equal to or less than 5 mN/m, further preferably equal to or less than 3 mN/m, particularly preferably equal to or less than 2 mN/m. The lower limit is more than 0 mN/m.

If the interfacial tension $r_{1/2}$ is beyond the above range, the stability of the emulsion to be formed by emulsifying the resulting system separated into two phases as described later decreases, and the resulting emulsion has a broad droplet size distribution. The particle diameter distribution of the resulting porous ether cellulose derivative microparticles is deemed to be broad for this reason.

The viscosity ratio of the two phases formed by phase separation affects the average particle diameter and the PDI of the resulting porous ether cellulose derivative microparticles. A preferable range of the viscosity ratio is represented by a lower limit of equal to or more than 0.1, more preferably equal to or more than 0.2, further preferably equal to or more than 0.3, more preferably equal to or more than 0.5, remarkably preferably equal to or more than 0.8. The upper limit of the viscosity ratio is preferably equal to or less than 10, more preferably equal to or less than 5, further preferably equal to or less than 3, particularly preferably equal to or less than 1.5, remarkably preferably equal to or less than 1.2. The viscosity ratio of the two phases here is defined as "the viscosity of the ether cellulose derivative (A) solution phase"/"the viscosity of the polymer (B) solution phase" under an actual temperature condition.

It is not preferable that the viscosity ratio between the two phases be larger or smaller than the above range because it becomes difficult to apply sufficient shearing force to the system at the time of emulsification described later, which hinders formation of an emulsion including uniform droplets.

A polymer solution that undergoes phase separation is prepared under the conditions under which phase separation occurs determined as described above, the solution is mixed to form an emulsion, which is used in the step of producing porous ether cellulose derivative microparticles.

In the method for producing a porous ether cellulose derivative microparticle according to the embodiment of the present invention, the steps of preparing a polymer solution, forming an emulsion, and forming a microparticle are carried out in a common reaction vessel.

In the step of preparing a polymer solution, the ether cellulose derivative (A), the polymer (B), and the alcohol solvent (C) that have arbitrary compositions and show the phase separation property are mixed, and the ether cellulose derivative (A) and the polymer (B) are completely dissolved in the alcohol solvent (C) to provide the polymer solution that undergoes phase separation. The temperature at which this step is carried out is equal to or higher than a temperature at which the ether cellulose derivative (A) and the polymer (B) is dissolved in the alcohol solvent (C). Preferable temperatures vary depending on the types of the polymers and cannot be uniquely determined. In view of industrial feasibility, temperatures of 0 to 300°C are preferable. The lower limit of the temperature range is preferably equal to or higher than 10°C, more preferably equal to or higher than 20°C, further preferably equal to or higher than 30°C, particularly preferably equal to or higher than 40°C, in view of the solubility of the ether cellulose derivative (A) and the polymer (B) and industrial feasibility. Th& upper limit of the temperature range is preferably equal to or lower than 250°C, more preferably equal to or lower than 225°C, further preferably equal to or lower than 200°C, in view of suppressing decomposition of the ether cellulose derivative (A) and the polymer (B).

The pressure in the step of preparing a polymer solution is in the range of atmospheric pressure to 100 atm (10.1 MPa) in view of industrial feasibility. Although depending on the saturated vapor pressure of the polymer solution at the temperature in this step, a preferable upper limit is equal to or less than 75 atm (7.5 MPa), further preferably equal to or less than 50 atm (5.0 MPa), particularly preferably equal to or less than 30 atm (3.0 MPa). Industrial working at a pressure beyond this range is not preferable because special and complicated pressure resistant designs of apparatuses and piping are required.

A preferable lower limit is equal to or higher than the saturated vapor pressure of the polymer solution at the temperature in the step of forming an emulsion in order to suppress boiling or excessive evaporation of the polymer solution.

The step of preparing a polymer solution is preferably carried out in an inert gas atmosphere. Nitrogen, helium, argon, or carbon dioxide is preferably used as the inert gas, and nitrogen or argon is more preferable.

The polymer solution obtained in the above step is stirred and mixed to form an emulsion. The temperature in the step of forming an emulsion is not particularly limited as long as the temperature is equal to or higher than a temperature at which the ether cellulose derivative (A) and the polymer (B) are dissolved in the alcohol solvent (C), but temperatures of 0 to 300°C are preferable in view of industrial feasibility. The lower limit of the temperature range cannot be uniquely determined because an appropriate temperature varies depending on the types of the ether cellulose derivative (A), the polymer (B), and the alcohol solvent (C). The lower limit is not particularly limited as long as the temperature is higher than the precipitation temperature of the ether cellulose derivative (A), and the temperature can be controlled in a similar manner as in the step of preparing a polymer solution.

The pressure and atmosphere in the step of forming an emulsion can also be controlled in a similar manner as in the step of preparing a polymer solution.

A known stirring method can be used to obtain shearing force sufficient to form the emulsion. Examples of the method include liquid phase stirring using stirring blades, stirring in a continuous twin mixer, mixing in a homogenizer, and ultrasonic irradiation.

In the case of the stirring using stirring blades, although depending on the shape of the stirring blades, the stirring rate is preferably 50 to 1,200 rpm, more preferably 100 to 1,000 rpm, further preferably 200 to 800 rpm, particularly preferably 300 to 600 rpm.

Examples of the stirring blades include propellers, paddles, flat paddles, turbines, double cones, single cones, single ribbons, double ribbons, screws, and helical ribbons, but the stirring blades are not particularly limited to these examples as long as the stirring blades can apply sufficient shearing force to the system. Also, a baffle plate or the like may be disposed in the vessel to perform efficient stirring.

A stirrer is not necessarily used to form the emulsion. An apparatus such as an emulsifier and a disperser may be used instead. Specific examples thereof include a homogenizer (manufactured by IKA Japan K.K.), Polytron (manufactured by Kinematica AG), a batch-type emulsifier such as T.K. Autohomomixer (manufactured by Tokushu Kika Kogyo Co., Ltd.), Ebara Milder (manufactured by Ebara Corporation), T.K. Filmics and T.K. Pipeline Homomixer (manufactured by Tokushu Kika Kogyo Co., Ltd.), a colloid mill (manufactured by Nihonseiki Kaisha Ltd.), Slasher and Trigonal Wet Milling Machine (manufactured by Nippon Coke & Engineering Company, Ltd.), an ultrasonic homogenizer, and a static mixer.

In the emulsion obtained as described above, the ether cellulose derivative (A) solution phase serves as the disperse phase, and the polymer (B) solution phase serves as the continuous phase. In the present embodiment, such an emulsion is successively subjected to the step of forming microparticles by precipitating microparticles. Specifically, bringing the poor solvent (D) into contact with the emulsion formed in the above step precipitates the porous ether cellulose derivative microparticle having a size that depends on the droplet size of the emulsion.

The temperature in the reaction vessel when the poor solvent (D) is brought into contact is not particularly limited as long as the temperature is equal to or higher than a temperature at which the ether cellulose derivative (A) and the polymer (B) are dissolved in the alcohol solvent (C), but temperatures of 0 to 300°C are preferable in view of industrial feasibility. The lower limit of the temperature range cannot be uniquely determined because an appropriate temperature varies depending on the types of the ether cellulose derivative (A) and the alcohol solvent (C). The lower limit is not particularly limited as long as the temperature is higher than the precipitation temperature of the ether cellulose derivative (A). A specific example of the lower limit of the temperature range is preferably equal to or more than 10°C, more preferably equal to or more than 30°C, further preferably equal to or more than 40°C, particularly preferably equal to or more than 50°C. The upper limit of the temperature range is preferably low enough to prevent the poor solvent (D) from reaching its boiling point at the pressure in the vessel when the poor solvent (D) is brought into contact. A specific example thereof is preferably equal to or less than 250°C, more preferably equal to or less than 225°C, further preferably equal to or less than 200°C.

The contact between the poor solvent (D) and the emulsion may be achieved either by putting the emulsion in the poor solvent (D) or putting the poor solvent (D) in the emulsion, but putting the poor solvent (D) in the emulsion is more preferable.

In this case, the method for putting in the poor solvent (D) is not particularly limited as long as the porous ether cellulose derivative microparticles according to the embodiment of the present invention are obtained. Any of continuous dropping, split addition, and batch addition may be employed. To prevent the emulsion from coagulating, fusing, or uniting when adding the poor solvent (D) and prevent the resulting porous ether cellulose derivative microparticles from being deformed and having a large PDI, continuous dropping or split dropping is preferably employed, and continuous dropping is most preferable in view of industrial efficiency.

The time period that the poor solvent (D) is added is preferably equal to or more than 5 minutes, more preferably equal to or more than 10 minutes, further preferably equal to or more than 30 minutes. Its upper limit is preferably equal to or less than 50 hours, more preferably equal to or less than 10 hours, further preferably equal to or less than 5 hours, particularly preferably equal to or less than 2 hours. Addition of the poor solvent (D) within this range of the time period suppresses coagulation of the particles when the porous ether cellulose derivative microparticles are precipitated from the emulsion, and microparticles having a high sphericity and a small PDI can be obtained.

Although depending on the amount of the poor solvent (D) to be added, it is not preferable that the time period that the poor solvent (D) is added be below the above range because the dropping rate of the poor solvent (D) increases, which decreases the efficiency of stirring and mixing the poor solvent (D) in the vessel. Also, it is not practically preferable that the time period that the poor solvent (D) is added be beyond the above range because the time cycle is elongated.

Addition of the poor solvent (D) over a time period shorter than the above range may decrease the sphericity or increase the PDI of the resulting porous ether cellulose derivative microparticles due to coagulating and fusing, or uniting in the emulsion. Also, a time period longer than the above range is industrially disadvantageous.

The optimum amount of the poor solvent (D) to be added varies according to the state of the emulsion varying depending on the molecular weight of the polymer (B) and the solubility of the ether cellulose derivative (A) in the alcohol solvent (C), but generally the amount is preferably 10 to 1,000 parts by mass relative to 100 parts by mass of the emulsion. Its upper limit is more preferably equal to or less than 500 parts by mass, further preferably equal to or less than 300 parts by mass, particularly preferably equal to or less than 200 parts by mass, most preferably equal to or less than 150 parts by mass. Its lower limit is preferably equal to or more than 10 parts by mass, further preferably equal to or more than 50 parts by mass.

The time period that the poor solvent (D) is in contact with the emulsion is long enough to sufficiently complete precipitation of the microparticles. To sufficiently develop the porous structure of the porous ether cellulose derivative microparticles by self-organization, the time period is preferably equal to or more than 5 minutes, more preferably equal to or more than 10 minutes, further preferably equal to or more than 20 minutes, most preferably equal to or more than 30 minutes after addition of the poor solvent (D) has been completed. To obtain efficient productivity, the upper limit is preferably equal to or less than 50 hours, more preferably equal to or less than 10 hours, further preferably equal to or less than 5 hours, particularly preferably equal to or less than 4 hours, most preferably equal to or less than 2 hours after addition of the poor solvent (D) has been completed.

From the dispersion of the porous ether cellulose derivative microparticles produced in this way, the porous ether cellulose derivative microparticles can be collected by solid-liquid separation by a commonly known method, such as filtration, filtration under reduced pressure, pressure filtration, centrifugation, centrifugal filtration, and spray drying.

The microparticles obtained by solid-liquid separation is purified by removing impurities adhering to or contained in the microparticles by washing with a solvent or the like as appropriate.

The solvent that can be used for the washing is preferably a solvent that contributes to removal of the polymer (B) and the alcohol solvent (C), which are highly likely to remain as impurities, and does not affect the porous structure of the resulting porous ether cellulose derivative microparticles. The poor solvent (D) can be used as such a solvent for washing.

The ether cellulose derivative (A) that can be used in the present producing method is not particularly limited as long as the porous ether cellulose derivative microparticles according to the embodiment of the present invention can be produced, but use of an ether cellulose derivative (A) having a degree of crystallinity of equal to or more than 2% is preferable. The degree of crystallinity of the ether cellulose derivative (A) is more preferably equal to or more than 3%, further preferably equal to or more than 3.5%, particularly preferably equal to or more than 4%, remarkably preferably equal to or more than 4.5%, most preferably equal to or more than 5%. Using such an ether cellulose derivative (A) provides porous ether cellulose derivative microparticles having a degree of crystallinity of equal to or more than 1%. The upper limit of the degree of crystallinity of the ether cellulose derivative (A) is preferably selected appropriately depending on the required solubility of the resulting porous ether cellulose derivative microparticles. For example, the upper limit is preferably equal to or less than 80%, more preferably equal to or less than 60%, further preferably equal to or less than 40%, particularly preferably equal to or less than 30%, remarkably preferably equal to or less than 20%, most preferably equal to or less than 15%. Making the degree of crystallinity of the ether cellulose derivative (A) be within the above range improves the solubility of the resulting porous ether cellulose derivative microparticles in various solvents and allows the microparticles to show solubility in a wide range of solvents, so that the range of the applications of the microparticles according to the embodiment of the present invention is broadened.

The degree of crystallinity of the ether cellulose derivative (A) can be calculated from a diffraction pattern measured by X-ray powder diffraction in a similar manner as for the degree of crystallinity of the porous ether cellulose derivative microparticles.

In the present producing method, the ether cellulose derivative described above can be used as the ether cellulose derivative (A) to produce the porous ether cellulose derivative microparticles.

In the present method, it is possible to recycle, that is, to use again, the alcohol solvent (C) and the polymer (B) separated in the step of solid-liquid separation performed when obtaining the particles.

The solvent separated in the step of solid-liquid separation is a mixture of the polymer (B), the alcohol solvent (C), and the poor solvent (D). The poor solvent (D) is removed from the solvent to recycle the mixture as the solvent for forming an emulsion. The poor solvent (D) can be removed by a known method. Specific examples thereof include simple distillation, distillation under reduced pressure, rectification, thin-film distillation, extraction, and membrane separation. Simple distillation, distillation under reduced pressure, or rectification is preferable.

A distillation operation such as simple distillation and distillation under reduced pressure is performed preferably in a state with as little oxygen as possible, more preferably in an inert atmosphere, to reduce the risk that thermal decomposition of the polymer (B) and the alcohol solvent (C) may be promoted due to heat applied to the system. Specifically, the operation is performed preferably in a nitrogen, helium, argon, or carbon dioxide atmosphere. A phenolic compound may be added as an antioxidant when performing the distillation operation.

When recycling the solvent and the like, the poor solvent (D) is preferably removed as completely as possible. Specifically, it is desirable that the amount of the remaining poor solvent (D) in the solvent after the poor solvent (D) has been removed be equal to or less than 20 mass%, preferably equal to or less than 10 mass%, more preferably equal to or less than 5 mass%, particularly preferably equal to or less than 3 mass%, remarkably preferably equal to or less than 1 mass%, relative to the total amount of the alcohol solvent (C) and the polymer (B). It is not preferable that the amount is beyond this range because this situation results in a lower sphericity and a larger PDI of porous ether cellulose derivative microparticles to be obtained when the solvent is recycled as the solvent for forming an emulsion. The amount of the poor solvent (D) in the recycled solvent can be measured by a known method, such as gas chromatography and the Karl Fischer's method.

Since the alcohol solvent (C) and the polymer (B) may also be removed in actuality during the operation of removing the poor solvent (D), it is preferable to adjust the composition as appropriate when the collected solvent is reused.

Since the porous ether cellulose derivative microparticles according to the embodiment of the present invention have pores both on the surface of and inside the particles and have such a surface pore size that allows a target substance to be incorporated from the openings on the surface of the particles into the particles and retained as described above, the microparticles can be suitably used as a carrier that selectively supports a target substance or as an adsorbent that selectively adsorbs a target substance in a medium.

In addition, since the ether cellulose derivative constituting the porous ether cellulose derivative microparticles according to the embodiment of the present invention has unique dissolution behavior that is not observed for other synthetic polymers, the porous ether cellulose derivative microparticles according to the embodiment of the present invention can be used as a concentrating agent in such a way that, for example, the microparticles adsorb and selectively collect a target component in a medium, and only the microparticles are selectively dissolved to extract the target component.

A physiologically-inactive active ingredient can be used as the target substance or target component, and the porous ether cellulose derivative microparticles according to the embodiment of the present invention can be used as a sustained-release base material of the physiologically-inactive active ingredient that incorporates the physiologically-inactive active ingredient into the microparticles and then sustainedly releases the physiologically-inactive active ingredient from the inside to the outside of the microparticles.

Also, since the porous ether cellulose derivative microparticles according to the embodiment of the present invention can be used as a base material that incorporates a physiologically-inactive active ingredient into the microparticles and then sustainedly releases the physiologically-inactive active ingredient from the inside to the outside of the microparticles, composite microparticles including the porous ether cellulose derivative microparticles according to the embodiment of the present invention and a physiologically-inactive active ingredient can have a property of sustainedly releasing the physiologically-inactive active ingredient.

The composite microparticles may be produced by compositing the two materials, which are the porous ether cellulose derivative microparticles according to the embodiment of the present invention and the physiologically-inactive active ingredient, or compositing the two materials with additives as other components such as excipients and binders.

Furthermore, since the porous ether cellulose derivative microparticles according to the embodiment of the present invention have such a particle diameter that the microparticles are suitably handled practically as a powder, the microparticles can be suitably used as a carrier, an adsorbent, a concentrating agent, or a sustained-release base material of a physiologically-inactive active ingredient in various applications.

In particular, since the porous ether cellulose derivative microparticles according to the embodiment of the present invention have a good oil absorption capacity, the microparticles can be used for collecting oil that has leaked to the environment. After the oil is collected, only the collected oil can be selectively extracted by treating the microparticles with a solvent that selectively dissolves only the ether cellulose derivative. Also, since the ether cellulose derivative is a carbon-neutral material, the porous ether cellulose derivative microparticles according to the embodiment of the present invention that have been used for selectively collecting the oil are suitably used as a solid fuel.

The physiologically-inactive substance in the embodiment of the present invention is a substance other than physiologically active substances contained in pharmaceuticals for medical use or health supplementary foods (supplements) that have effects on the human body. That is, substances other than substances that show pharmacological activity in the case of pharmaceuticals and that have a function of maintaining and improving health of the body in the case of health supplementary foods are meant. The pharmaceuticals or health supplementary foods here include materials under development with the goal of obtaining approval as pharmaceuticals or the goal of sale as health supple-

EP 3 369 749 A1

mentary foods in the future.

The physiologically-inactive active ingredient in the embodiment of the present invention is not particularly limited as long as the ingredient is the physiologically-inactive active ingredient as described above. Specific examples of the physiologically-inactive active ingredient include perfumes, sweeteners, acidulants, antioxidants, preservatives, disinfectants, coloring agents (coloring matters, pigments, dyes, inks, and paints), agricultural chemicals, fertilizers, repellents against or attractants for bugs and animals, fungicides, sterilants, germicides, antimicrobial agents, antibacterial agents, preservative agents, antiseptic agents, deodorizers, and lubricating oils.

Examples of the sweeteners include sugar, glucose, fructose, isomerized liquid sugar, fructooligosaccharides, aspartame, sorbitol, and stevia. Examples of the acidulants include adipic acid, citric acid, gluconic acid, succinic acid, d-tartaric acid, lactic acid, and dl-malic acid. Examples of the antioxidants include L-ascorbic acid, erythorbic acid, and rutin. Examples of the preservatives include pectin digests, benzoic acid, sorbic acid, p-hydroxybenzoic acid esters, and potassium sorbate. Examples of the disinfectants include bleaching powder, hydrogen peroxide, and hypochlorous acid. Examples of the coloring agents include the red cabbage color, the grape skin color, the elderberry color, caramel, the gardenia color, the corn color, the saffron color, and carotene. One of these substances may be used singly, or a mixture of equal to or more than two substances mixed at an arbitrary ratio may be used.

In the embodiment of the present invention, the sterilants, the germicides, the antimicrobial agents, the antibacterial agents, the preservative agents, the antiseptic agents, and the fungicides are defined as follows. The sterilants are substances that kill and remove all microorganisms. The statement that the substances kill and remove all microorganisms here means, for example, an action of reducing the probability for the existence of microorganisms to equal to or less than one millionth. The germicides are substances that kill microorganisms such as bacteria and viruses regardless of the magnitude of the effects. The antimicrobial agents are substances that reduce the number of microorganisms in a limited space. The antibacterial agents are substances that prevent proliferation of bacteria. The preservative agents are substances that hinder or prevent proliferation of microorganisms or substances that suppress adhesion of microorganisms. The antiseptic agents are substances that reduce pathogenic microorganisms adhering to an object or a living body to a harmless level or counteract the toxicity to prevent infection by causing the microorganisms to lose their infectivity. The fungicides are substances that hinder or prevent growth or proliferation of molds.

Examples of the substances serving as the above germicides, antimicrobial agents, antibacterial agents, preservative agents, antiseptic agents, and fungicides include aldehydes such as glutaraldehyde, glyoxal, $\alpha$-bromocinnamaldehyde, and formalin; alcohols such as ethanol, isopropanol, and benzyl alcohol; iodine compounds such as iodine tincture, iodophenols, iodoform, iodophors, and povidone-iodine; chlorine compounds such as hypochlorite salts, chlorine dioxide, 1,3-dichloro-5,5-dimethylhydantoin, p-toluenesulfonchloramide sodium salt, and polychloroisocyanurate salts; peroxides such as hydrogen peroxide, sodium percarbonate, sodium peroxypyrophosphate, and peracetic acid; phenols such as phenol, Lysol, cresols, xylenols, parachlorometaxylenol, and diphenyl compounds; acids such as boric acid, benzoic acid, salicylic acid, dehydroacetic acid, sorbic acid, and salts thereof; amide compounds such as trichlorocarbanilides, halocarban, and tribromsalan; and biguanides such as chlorhexidine gluconate, chlorhexidine hydrochloride, and polyhexamethylenebiguanide. Other examples include quaternary ammonium salts such as benzalkonium chloride, benzethonium chloride, cetylpyridinium chloride, and methylbenzethonium chloride and surfactants such as sodium lauryl sulfate, alkyldiaminoethylglycinate salts, and betaine. One of these substances may be used singly, or a mixture of equal to or more than two substances mixed at an arbitrary ratio may be used.

In particular, the porous ether cellulose derivative microparticles according to the embodiment of the present invention can be suitably used as a sustained-release base material of a perfume. If a perfume is supported on the porous ether cellulose derivative microparticles according to the embodiment of the present invention, the perfume is sustainedly released, so that the microparticles can be used as a perfume agent that shows a lingering property over time.

The perfume is not particularly limited as long as composite microparticles obtained by causing the porous ether cellulose derivative microparticles according to the embodiment of the present invention to support the perfume show a sustained perfume property. Examples of the perfume include substances selected from hydrocarbons, alcohols, phenols, esters, carbonates, aldehydes, ketones, acetals, ethers, nitriles, carboxylic acids, lactones, and other natural essential oils and natural extracts.

Specific examples of the perfume include, as the hydrocarbons, limonene, $\alpha$-pinene, $\beta$-pinene, terpinene, cedrene, longifolene, and valencene.

Examples of the alcohols include citronellol, geraniol, terpineol, dihydromyrcenol, ethyllinalool, farnesol, nerolidol, cis-3-hexenol, cedrol, menthol, borneol, phenethyl alcohol, benzyl alcohol, dimethylbenzylcarbinol, phenylethyldimethylcarbinol, phenylhexanol, 2,2,6-trimethylcyclohexyl-3-hexanol, and 1-(2-t-butylcyclohexyloxy)-2-butanol.

Examples of the phenols include guaiacol, eugenol, isoeugenol, and vanillin.

Examples of the esters include formate esters, acetate esters, propionate esters, butyrate esters, nonenoate esters, benzoate esters, cinnamate esters, salicylate esters, brassylate esters, tiglate esters, jasmonate esters, glycidate esters, and anthranilate esters. Examples of the formate esters include linalyl formate, citronellyl formate, and geranyl formate. Examples of the acetate esters include n-hexyl acetate, cis-3-hexenyl acetate, linalyl acetate, citronellyl acetate, geranyl acetate, neryl acetate, terpinyl acetate, nopyl acetate, bornyl acetate, isobornyl acetate, o-(t-butyl)cyclohexyl acetate, p-(t-butyl)cyclohexyl acetate, tricyclodecenyl acetate, benzyl acetate, styrallyl acetate, cinnamyl acetate, dimethylbenzylcarbinyl acetate, phenylethyl phenylacetate, and 3-pentyltetrahydropyran-4-yl acetate. Examples of the propionate esters include citronellyl propionate, tricyclodecenyl propionate, allylcyclohexyl propionate, ethyl-2-cyclohexyl propionate, and benzyl propionate. Examples of the butyrate esters include citronellyl butyrate, dimethylbenzylcarbinyl n-butyrate, and tricyclodecenyl isobutyrate. Examples of the nonenoate esters include methyl 2-nonenoate, ethyl 2-nonenoate, and ethyl 3-nonenoate. Examples of the benzoate esters include methyl benzoate, benzyl benzoate, and 3,6-dimethyl benzoate. Examples of the cinnamate esters include methyl cinnamate and benzyl cinnamate. Examples of the salicylate esters include methyl salicylate, n-hexyl salicylate, cis-3-hexenyl salicylate, cyclohexyl salicylate, and benzyl salicylate. Examples of the brassylate esters include ethylene brassylate. Examples of the tiglate esters include geranyl tiglate, 1-hexyl tiglate, and cis-3-hexenyl tiglate. Examples of the jasmonate esters include methyl jasmonate and methyl dihydrojasmonate. Examples of the glycidate esters include methyl 2,4-dihydroxy-ethylmethylphenylglycidate and 4-methylphenylethyl glycidate. Examples of the anthranilate esters include methyl anthranilate, ethyl anthranilate, and dimethyl anthranilate.

Examples of the carbonates include methyl-cyclooctyl carbonate (JASMACYCLAT (trade name) manufactured by Kao Corporation), ethyl-2-(tert-butyl)cyclohexyl carbonate (FLORAMAT (trade name) manufactured by Kao Corporation), methyl-3-cyclooctenyl carbonate (Violiff (trade name) manufactured by International Flavors & Fragrances Inc.), ethyl-cyclooctyl carbonate, methyl-trans- 3,3,5 -trimethylcyclohexyl carbonate, ethyl-trans-3,3,5 -trimethylcyclohexyl carbonate, methyl-cis-3,3,5-trimethylcyclohexyl carbonate, methyl-1-ethynylcyclohexyl carbonate, methyl-2-(tert-butyl)cyclohexyl carbonate, ethyl-2-(tert-butyl)cyclohexyl carbonate, methyl-4-(tert-butyl)cyclohexyl carbonate, ethyl-4-(tert-butyl)cyclohexyl carbonate, methyl-4-cyclooctenyl carbonate, and ethyl-4-cyclooctenyl carbonate.

Examples of the aldehydes include n-octanal, n-decanal, n-dodecanal, 2-methylundecanal, 10-undecenal, citronellal, citral, hydroxycitronellal, benzaldehyde, phenylacetaldehyde, phenylpropylaldehyde, cinnamic aldehyde, dimethyltetrahydrobenzaldehyde, 2-cyclohexylpropanal, p-(t-butyl)-$\alpha$-methylhydrocinnamic aldehyde, p-isopropyl-$\alpha$-methylhydrocinnamic aldehyde, p-ethyl-$\alpha,\alpha$-dimethylhydrocinnamic aldehyde, $\alpha$-amylcinnamic aldehyde, $\alpha$-hexylcinnamic aldehyde, heliotropin, and $\alpha$-methyl-3,4-methylenedioxyhydrocinnamic aldehyde.

Examples of the ketones include $\alpha$-ionone, $\beta$-ionone, $\gamma$-ionone, $\alpha$-methylionone, $\beta$-methylionone, $\gamma$-methylionone, methylheptenone, 4-methylene-3,5,6,6-tetramethyl-2-heptanone, amylcyclopentanone, 3-methyl-2-(cis-2-penten-1-yl)-2-cyclopenten-1-one, methylcyclopentenolone, rose ketones, carvone, menthone, camphor, acetylcedrene, isolongifolanone, nootkatone, benzylacetone, anisylacetone, methyl $\beta$-naphthyl ketone, 2,5-dimethyl-4-hydroxy-3(2H)-furanone, maltol, muscone, civetone, cyclopentadecanone, and cyclohexadecanone.

Examples of the acetals include formaldehyde cyclododecyl ethyl acetal, acetaldehyde ethyl phenyl propyl acetal, citral diethyl acetal, phenylacetaldehyde glycerin acetal, and ethylacetoacetate ethylene glycol acetal.

Examples of the ethers include cedryl methyl ether, anethole, $\beta$-naphthyl methyl ether, $\beta$-naphthyl ethyl ether, limonene oxide, rose oxide, nerol oxide, 1,8-cineole, rosefuran, and decahydro-3a,6,6,9a-tetramethylnaphtho[2.1-b]furan.

Examples of the nitriles include geranylnitrile, citronellylnitrile, and dodecanenitrile.

Examples of the carboxylic acids include benzoic acid, phenylacetic acid, cinnamic acid, hydrocinnamic acid, butyric acid, and 2-hexenoic acid.

Examples of the lactones include $\gamma$-decalactone, $\delta$-decalactone, $\gamma$-valerolactone, $\gamma$-nonalactone, $\gamma$-undecalactone, $\delta$-hexalactone, $\gamma$-jasmolactone, whisky lactone, coumarin, cyclopentadecanolide, cyclohexadecanolide, ambrettolide, ethylene brassylate, 11-oxahexadecanolide, and butylidenephthalide.

Examples of the natural essential oils and natural extracts include orange, lemon, lime, bergamot, vanilla, mandarin, peppermint, spearmint, lavender, chamomile, rosemary, eucalyptus, sage, basil, rose, rock rose, geranium, jasmine, ylang-ylang, anise, clove, ginger, nutmeg, cardamon, cedar, hinoki, vetiver, patchouli, lemon grass, and labdanum oils and extracts.

One of these perfumes may be used singly, or a mixture of equal to or more than two perfumes mixed at an arbitrary ratio may be used.

Also, in the embodiment of the present invention, an active ingredient that shows physiological activity, that is, a physiologically active substance, can be used as the target substance or target component. By providing composite microparticles including the porous ether cellulose derivative microparticles obtained in the embodiment of the present invention and a physiologically active substance, composite microparticles for preparation (hereinafter also referred to simply as particles for preparation) that show properties of supporting the physiologically active substance and discharging and sustainedly releasing the physiologically active substance supported depending on the purpose can be obtained.

In the embodiment of the present invention, the porous ether cellulose derivative microparticles used for the particles for preparation may contain components other than the ether cellulose derivative as long as the objects of the present invention are not impaired. In this case, the content of the ether cellulose derivative is preferably equal to or more than 50 mass%, more preferably equal to or more than 70 mass%, further preferably equal to or more than 80 mass%, particularly preferably equal to or more than 90 mass%, remarkably preferably equal to or more than 95 mass%. It is preferable that approximately no component other than the ether cellulose derivative be contained. The upper limit of the content of the ether cellulose derivative is 100 mass%.

In the case where the content of the ether cellulose derivative in the porous ether cellulose derivative microparticles used for particles for preparation is less than 100 mass%, common pharmaceutical additives (such as excipients, disintegrators, desiccants, stabilizers, binding agents, and lubricants) may be contained as components other than the ether cellulose derivative.

The particles for preparation in the embodiment of the present invention are provided by causing a physiologically active substance described later to be supported on the porous ether cellulose derivative microparticles according to the embodiment of the present invention having the surface pores and the inner pores.

Also, a preparation can be obtained using the particles for preparation containing the porous ether cellulose derivative microparticles according to the embodiment of the present invention. The preparation in the embodiment of the present invention includes all preparations, including solid preparations and liquid preparations, used for pharmaceuticals and health supplementary foods (supplements) containing physiologically active substances. Examples of the solid preparations include powder preparations, tablets, capsule preparations (including soft capsules and microcapsules), powdered drugs, granules, pills, troches, dry syrups, film preparations, nasal preparations, inhalant, freeze-dried preparations for injection, and transdermal patches. Examples of the liquid preparations include suspensions, lotions, syrups, ointments, aerosols, creams, gels, elixirs, suspensions, emulsions, and pastes.

The powder preparations in the embodiment of the present invention are the powder of the particles for preparation according to the embodiment of the present invention. The powder preparations can contain additives as appropriate, and the powder preparations may be mixed powders containing the particles for preparation and additives.

In the embodiment of the present invention, the tablets are solid preparations having certain shapes that are orally administered. The tablets can be made by compression-molding the particles for preparation, and additives can be added as appropriate at the time of compression molding. Examples of the additives that can be added to the tablets include excipients, binding agents, and disintegrators. Examples of the tablets include orally disintegrating tablets, chewable tablets, and dispersible tablets. The particles for preparation according to the embodiment of the present invention can be particularly suitably used for orally disintegrating tablets.

In the embodiment of the present invention, the granules are granulated preparations made by forming the particles for preparation into granules. Additives, such as excipients, binding agents, and disintegrators, can be added and mixed in the middle of, before, or after granulation. Although depending on the average particle diameter of the ether cellulose derivative microparticles according to the embodiment of the present invention, the average particle diameter of the granules is, for example, 500 to 1,500 $\mu$m. Among the granules, when the total amount of a type of granules passes through a No. 18 sieve (850 $\mu$m) and equal to or less than 10% of the total amount remains on a No. 30 sieve (500 $\mu$m), the granules are referred to as fine granules. Although depending on the average particle diameter of the ether cellulose derivative microparticles according to the embodiment of the present invention, the average particle diameter of the fine granules is, for example, 75 to 500 $\mu$m.

In the embodiment of the present invention, the powdered drugs are granulated preparations made by forming the particles for preparation into powders. The granulation can be performed after mixing the particles for preparation and additives, such as excipients, binding agents, and disintegrators, and making the mixture homogeneous. The powdered drugs are such preparations that the total amount of the preparations passes through a No. 18 sieve (850 $\mu$m) and that equal to or less than 5% of the total amount remains on a No. 30 sieve (500 $\mu$m). Although depending on the average particle diameter of the ether cellulose derivative microparticles according to the embodiment of the present invention, the average particle diameter of the powdered drugs is, for example, 10 to 500 $\mu$m.

The average particle diameters of the granules, the fine granules, and the powdered drugs can be determined as the arithmetic mean of particle diameters measured using images observed under a scanning electron microscope in a similar manner as the method for calculating the average particle diameter of the porous ether cellulose derivative microparticles.

In the embodiment of the present invention, the pills are preparations made by adding additives to the particles for preparation and then forming the mixture into a spherical shape. Examples of the additives that can be added to the pills include excipients, binding agents, and disintegrators.

The capsule preparations are provided by directly charging the above powder preparations, granules, fine granules, or powdered drugs into capsules or by mixing the above powder preparations, granules, fine granules, or powdered drugs with lactose, mannitol, or the like and then encapsulating the mixtures in a capsule base. Examples of the capsule base include gelatin.

The solid preparations include preparations that float in gastric juice and release physiologically active substances over a long period of time. The solid preparations that float may be provided by forming the particles for preparation according to the embodiment of the present invention into granules or tablets by a conventional method or may be capsule preparations provided by charging the particles into capsules. The nasal preparations include nasal preparations that release physiologically active substances over a long period of time. The nasal preparations can be used in the form of, for example, sprays or powder sprays.

The preparations obtained using the particles for preparation according to the embodiment of the present invention can be sustained-release preparations. The sustained-release preparations have adjusted release rates, release times, and release sites of physiologically active substances from the preparations. The sustained-release preparations include taste-masked preparations in which the release is slightly delayed to make the bitter taste of the physiologically active substances less sensible in the mouth.

The physiologically active substances contained in the preparations according to the embodiment of the present invention are substances contained in pharmaceuticals for medical use or health supplementary foods (supplements) that have effects on the human body. That is, substances that show pharmacological activity in the case of pharmaceuticals and substances that have a function of maintaining and improving health of the body in the case of health supplementary foods are employed. The pharmaceuticals or health supplementary foods here include materials under development with the goal of obtaining approval as pharmaceuticals or the goal of sale as health supplementary foods in the future.

The physiologically active substances can be appropriately selected depending on the purpose, and an example thereof is one or two or more components selected from the group consisting of nutritional health supplements, herbal medicines, antipyretic, analgesic, and anti-inflammatory drugs, psychotropic drugs, antianxiety drugs, antidepressants, sedative-hypnotic drugs, spasmolytic drugs, central nervous system acting drugs, brain metabolic stimulants, ameliorants of cerebral circulation, antiepileptics, sympathomimetic agents, digestive drugs, antacids, antiulcer drugs, antitussive expectorants, antiemetics, respiratory stimulants, bronchodilators, antiallergic drugs, agents for dental and oral use, antihistamines, cardiotonics, antiarrhythmic agents, diuretics, antihypertensives, vasoconstrictors, coronary vasodilators, peripheral vasodilators, antihyperlipidemics, cholagogues, therapeutic agents for chronic arterial occlusive diseases, antibiotics, chemotherapeutics, antidiabetic agents, drugs for osteoporosis, antirheumatic drugs, skeletal muscle relaxants, antispasmodics, hormone drugs, alkaloid narcotics, sulfa drugs, therapeutic agents for gout, anticoagulants, antineoplastic drugs, therapeutic agents for Alzheimer's disease, and antipruritics.

Among the above physiologically active substances, substances such as physiologically active substances that are required to be contained in the preparations at high contents, oily physiologically active substances, and physiologically active substances having low melting points particularly easily cause troubles with tableting such as poor fluidity and sticking (adhesion to the punch face) when compressed at the normal temperature. The fluidity of such physiologically active substances can be improved by compositing the substances with the porous ether cellulose derivative microparticles according to the embodiment of the present invention to form particles for preparation. Furthermore, the troubles with tableting can be ameliorated by compositing such physiologically active substances with the porous ether cellulose derivative microparticles according to the embodiment of the present invention to cause the microparticles to support the substances.

Examples of the health supplementary foods include lutein, folic acid, fatty acids (such as docosahexaenoic acid, arachidonic acid, and eicosapentaenoic acid), fruit or vegetable extracts, vitamin or mineral supplements, phosphatidyl serine, lipoic acid, melatonin, glucosamine, chondroitin, aloe vera, guggul, amino acids (such as glutamine, arginine, isoleucine, leucine, lysine, methionine, phenylalanine, threonine, tryptophan, and valine), green tea, lycopene, wholefood, plant nutrients, antioxidants, flavonoid ingredients in fruits, evening primrose oil, linseed oil, fish oil or marine animal oil, and probiotics.

Also, causing the porous ether cellulose derivative microparticles according to the embodiment of the present invention to support substances such as bitter physiologically active substances and physiologically active substances that require control of release achieves taste masking by slightly delayed release or taste masking without delaying the release, utilizing the property of the microparticles of being porous. Furthermore, in a preferable aspect, a sustained-release coating can be uniformly layered utilizing the property of the microparticles of being spherical with the particles for preparation that support physiologically active substances being core particles, which is of high utility value.

Furthermore, the porous ether cellulose derivative microparticles according to the embodiment of the present invention used for the particles for preparation exhibit lower hygroscopicity than the hygroscopicity of porous particles formed of inorganic substances. Generally, physiologically active substances that have got contact with hygroscopic additives are known to become unstable due to transferring of water from the additives. Since the porous ether cellulose derivative microparticles according to the embodiment of the present invention are less likely to support water, the resulting particles for preparation can be stably stored for a long period with unstable or deliquescent physiologically active substances being supported.

In a preferable aspect, the porous ether cellulose derivative microparticles according to the embodiment of the present invention have high sphericity and show extremely high fluidity. Hence, the resulting particles for preparation can be used for the purpose of not only improving the fluidity of physiologically active substances but also improving the fluidity of granules to be charged into capsules, granules to be tableted, and the like.

Furthermore, since the porous ether cellulose derivative microparticles according to the embodiment of the present invention have an ether cellulose derivative content of preferably equal to or more than 50 mass%, the microparticles exhibit a small surface energy and thus can favorably support hydrophobic or slightly-soluble physiologically active substances. Examples of a method for causing hydrophobic or slightly-soluble physiologically active substances to be supported include causing a physiologically active substance in an amorphous state to be supported on the ether cellulose derivative microparticles. Another examples is to cause a physiologically active substance in a form of a dispersion or solid solution in at least one selected from the group consisting of polymers, surfactants, oils, fats, waxes, fatty alcohols, and fatty acids to be supported on the ether cellulose derivative microparticles. Employing these methods improves the solubility of the porous ether cellulose derivative microparticles supporting physiologically active substances and improves bioavailability of the microparticles.

In the embodiment of the present invention, among esters of fatty acids and glycerin, esters that are in a liquid state at the normal temperature are referred to as oils, and esters that are in a solid state at the normal temperature are referred to as fats. Waxes in the embodiment of the present invention mean esters of fatty acids and monohydric or dihydric alcohols.

Causing the porous ether cellulose derivative microparticles according to the embodiment of the present invention to support a physiologically active substance means creating a state in which the physiologically active substance is adsorbed in or adheres to at least the inside of the pores of the porous ether cellulose derivative microparticles and/or a state in which the physiologically active substance covers the porous ether cellulose derivative microparticles. When producing such porous ether cellulose derivative microparticles supporting a physiologically active substance, the form of the physiologically active substance is not limited. The physiologically active substance can be added to the porous ether cellulose derivative microparticles as a solid and/or liquid.

In the particles for preparation including the porous ether cellulose derivative microparticles according to the embodiment of the present invention, the physiologically active substance is not necessarily supported on the porous ether cellulose derivative microparticles but may be contained in the particles for preparation independently of the porous ether cellulose derivative microparticles as long as targeted eluting ability and sustained-release ability can be obtained.

A physiologically active substance can be added in a solid state to the porous ether cellulose derivative microparticles by any method, and examples of the method include a method of mixing a fine physiologically active substance having been subjected to a powdering treatment with the porous ether cellulose derivative microparticles, a method of adding a physiologically active substance suspended in a solvent such as water, ethanol, and methanol or a mixed solution thereof to the porous ether cellulose derivative microparticles, and a method of adding a physiologically active substance formed into micelles using a surfactant or the like to the porous ether cellulose derivative microparticles.

A physiologically active substance can be added in a liquid state by any method, and examples of the method include a method of adding a melt of a physiologically active substance heated to a temperature equal to or higher than its melting point to the porous ether cellulose derivative microparticles, a method of adding a solution of a physiologically active substance in a solvent such as water, ethanol, and methanol or a mixed solution thereof to the porous ether cellulose derivative microparticles, a method of adding a solution of a physiologically active substance dissolved using a solubilizer such as a surfactant, a basic substance, and an acidic substance to the porous ether cellulose derivative microparticles, and a method of adding and mixing a melt produced by dissolving a physiologically active substance in a substance having a low melting point by applying heat to the porous ether cellulose derivative microparticles.

Among these methods, to increase the amount of a physiologically active substance to be supported in the fine pores of the porous ether cellulose derivative microparticles, a method of adding a physiologically active substance as a suspension or a solution and a method of reducing the surface tension of the suspension or solution before adding the suspension or solution are more preferable. Specific examples of the method include a method of adding a physiologically active substance after dissolving or suspending the substance in an organic solvent such as ethanol or in a mixed solution of water and an organic solvent such as ethanol, and a method of reducing the surface tension by adding a surfactant to the solution or suspension before adding the solution or suspension. Examples of preferable surfactants include lauryl sulfate salts.

To cause a physiologically active substance to be more strongly supported on the porous ether cellulose derivative microparticles, a solution containing the physiologically active substance to be added may further contain a water-soluble polymer. Examples of the water-soluble polymer include natural polymers such as gelatin and gum arabic; cellulose derivatives such as hydroxypropyl cellulose, hydroxypropyl methyl cellulose, methyl cellulose, and sodium carboxymethyl cellulose; and synthetic polymers such as polyvinylpyrrolidone, poly(vinyl alcohol), and poly(ethylene glycol).

A commonly-used mixing, granulating, or coating apparatus may be used to cause the porous ether cellulose derivative microparticles to support a physiologically active substance. Examples of the apparatus include mixers, extrusion granulators, rotating granulators, fluidized bed granulators, agitation granulators, vacuum emulsifiers, centrifuges, apparatuses for filtration under reduced pressure, and apparatuses for pressure filtration. To cause a physiologically active substance to be efficiently supported in the fine pores of the porous ether cellulose derivative microparticles, utilization of centrifugal force or application or reduction of pressure is preferable. As a simple supporting method, use of a fluidized bed granulator or an agitation granulator is preferable because the porous ether cellulose derivative microparticles supporting a physiologically active substance in the fine pores are easily obtained. Furthermore, in the case where a fluidized bed granulator is used, a granule-coating fluidized bed granulator equipped with Wurster (registered trademark) or a tangential spray device is more preferably used.

Another advantage of the ether cellulose derivative is that the derivative is a nonionic polymer and does not exhibit strong chemical interaction with physiologically active substances. Hence, the particles for preparation obtained in the embodiment of the present invention can release 100 mass% of a physiologically active substance supported, and incomplete elution that is often observed in the case where a physiologically active substance is supported on a commonly-used inorganic porous substance does not occur.

A sustained-release layer can be formed on the particles for preparation including the porous ether cellulose derivative microparticles according to the embodiment of the present invention. The sustained-release layer prevents a medium into which an ingredient is sustainedly released from permeating and getting contact with the outer surface of the physiologically active substance supported on the porous ether cellulose derivative microparticles to suppress release of the active ingredient from the particles for preparation. That is, the sustained-release layer has a function of slowing down the release rate of the physiologically active substance from the particles for preparation. The sustained-release layer can be formed by, for example, a method of filling the fine pores of the porous ether cellulose derivative microparticles with the sustained-release layer and/or a method of layering a coating of the sustained-release layer on the outer surface of the microparticles. The sustained-release layer may be formed after the porous ether cellulose derivative microparticles have supported a physiologically active substance. Alternatively, the sustained-release layer may be formed simultaneously with supporting of a physiologically active substance by mixing the physiologically active substance and a sustained-release agent when causing the porous ether cellulose derivative microparticles to support the physiologically active substance. In the case where a physiologically active substance is supported on the porous ether cellulose derivative microparticles before forming the sustained-release layer, the physiologically active substance is not necessarily in contact with the sustained-release layer, and an isolating layer can be formed therebetween.

The sustained-release agent constituting the sustained-release layer is preferably at least one selected from the group consisting of polymers, surfactants, oils, fats, waxes, fatty alcohols, and fatty acids.

The polymer used for the sustained-release layer is a compound including a repeating unit structure in the molecule, and examples thereof include a water-soluble polymer that swells to form the sustained-release layer on the microparticles and an organic or inorganic polymer that forms a water-insoluble coating on the microparticles. The organic polymers here are compounds including repeating units mainly made of carbon skeletons, and the inorganic polymers are compounds including repeating units mainly made of silicon skeletons.

Among these polymers, acrylic polymers and/or ethyl cellulose can be preferably used as the water-insoluble organic polymers, and calcium silicate, magnesium silicate, or talc can be preferably used as the water-insoluble inorganic polymers. Examples of the acrylic polymers include aminoalkyl methacrylate copolymers, methacrylate copolymers, ammonioalkyl methacrylate copolymers, ethyl acrylate-methyl methacrylate copolymers, and examples of articles on the market include "Eudragit (registered trademark)" E, "Eudragit (registered trademark)" L, "Eudragit (registered trademark)" S, "Eudragit (registered trademark)" RL, "Eudragit (registered trademark)" RS, "Eudragit (registered trademark)" NE, and "Eudragit (registered trademark)" FS (each manufactured by Evonik Industries AG). These polymers may be added in any of the powder, solution, and suspension forms.

The surfactants used for the sustained-release layer are substances having any low HLBs (hydrophile-lipophile balances) among substances that have structures including both hydrophilic groups and lipophilic groups in the molecules, and examples thereof include alkylallyl polyether alcohols, sucrose esters of fatty acids, polyoxyethylene alkyl ethers, polyoxyethylene hydrogenated castor oil, propylene glycol esters of fatty acids, lauryl sulfate salts, stearate salts, sorbitan esters of fatty acids, poly(ethylene glycol) esters of fatty acids, polyoxyethylene glycerol esters of fatty acids, glycerol esters of fatty acids, polyoxyethylene polyoxypropylene glycol, polyoxyethylene sorbitol esters of fatty acids, polyoxyethylene alkylallyl ethers, alkylallylsulfonate salts, coconut fatty acid diethanolamide, polyoxyethylene sorbitan esters of fatty acids, and mixtures thereof. In particular, sucrose esters of fatty acids are preferably used.

In the embodiment of the present invention, among esters of fatty acids and glycerin, esters that are in a liquid state at the normal temperature are referred to as oils, and esters that are in a solid state at the normal temperature are referred to as fats. Waxes in the embodiment of the present invention mean esters of fatty acids and monohydric or dihydric alcohols. Examples of the oils, fats, waxes, fatty alcohols, or fatty acids used for the sustained-release layer include linseed oil, tung oil, soybean oil, peanut oil, sesame oil, palm oil, butterfat, rapeseed oil, corn oil, carnauba wax, cottonseed oil, esterified corn oil, orange oil, almond oil, safflower oil fatty acid, tocopherol acetate, chamomile oil, hydrogenated soybean oil, stearic acid, lauric acid, safflower oil, hard fats, camellia oil, rice-bran oil, castor oil, coconut oil, olive oil, Vaseline, octyl decyl triglyceride, cocoa butter, medium-chain fatty acid triglycerides, squalane, oleic acid, clove oil, white beeswax, wheat germ oil, stearyl alcohol, silicones, and mixtures thereof.

In addition, the release time by the sustained-release layer can be adjusted by mixing a pharmaceutically acceptable water-soluble substance. The water-soluble substance is preferably compatible with the sustained-release layer, and examples thereof include triethyl citrate, triacetin, poly(ethylene glycol), polyvinylpyrrolidone, poly(vinyl alcohol), methyl cellulose, and sodium carboxymethyl cellulose. Poly(ethylene glycol) is particularly preferable, and an appropriate molecular weight can be selected depending on the purpose. The mixing ratio of the water-soluble substance contained in the sustained-release layer is not particularly limited and can be appropriately selected depending on the purpose.

A commonly-used mixing, granulating, or coating apparatus may be used to form the sustained-release layer on the surface of the porous ether cellulose derivative microparticles. Examples of such an apparatus include rotating granulators, fluidized bed granulators, agitation granulators, vacuum emulsifiers, centrifuges, apparatuses for filtration under reduced pressure, and apparatuses for pressure filtration. In the case where the sustained-release layer is formed by filling the fine pores of the porous ether cellulose derivative microparticles, an emulsifier or an agitation granulator can preferably be used. In the case where a coating of the sustained-release layer is layered on the surface of the porous ether cellulose derivative microparticles, a fluidized bed granulator or a tumbling bed granulator can preferably be used. Operation of these apparatuses can be performed under common conditions.

In the case where the porous ether cellulose derivative microparticles on the surface of which the coating of the sustained-release layer is layered (hereinafter also referred to simply as sustained-release coated microparticles) are provided, the sustained-release coated microparticles are provided by continuously repeating spraying of a solution or suspension containing a sustained-release agent and drying the microparticles while tumbling the porous ether cellulose derivative microparticles supporting a physiologically active substance in a wet granulator. Alternatively, the sustained-release coated microparticles are provided by continuously repeating spraying of a solution or suspension containing a sustained-release agent in which a physiologically active substance is dissolved or suspended and drying the microparticles while flowing the porous ether cellulose derivative microparticles in a wet granulator. The order can be appropriately selected depending on the type of the physiologically active substance or the like.

A structure in which the sustained-release layer is disposed on the particles for preparation containing a physiologically active substance as an active ingredient has been described above, but a similar sustained-release layer can be disposed in a similar manner on composite microparticles containing a physiologically-inactive substance as an active ingredient. Effects similar to the effects in the case where a physiologically active substance is used as an active ingredient can be obtained also in such a case. That is, a medium into which an ingredient is sustainedly released is prevented from permeating and getting contact with the outer surface of the active ingredient supported on the porous ether cellulose derivative microparticles, and the release rate of the active ingredient from the composite microparticles can be slowed down.

Also, the sustained-release property can be imparted to the particles for preparation by modifying the surface of the porous microparticles utilizing solubility of the porous ether cellulose derivative microparticles according to the embodiment of the present invention in a solvent without layering the coating of the sustained-release layer on the surface of the porous ether cellulose derivative microparticles.

Specific examples thereof include a method of performing a step of causing a physiologically active substance to be supported in a solvent that can dissolve the porous ether cellulose derivative or in a mixed solvent of a solvent that can dissolve the porous ether cellulose derivative and a solvent that cannot dissolve the porous ether cellulose derivative to modify the surface of the porous ether cellulose derivative microparticles to provide particles for preparation containing the physiologically active substance in the fine pores of the porous ether cellulose derivative microparticles; a method of performing a step of spraying a solvent that can dissolve the porous ether cellulose derivative or a mixed solvent of a solvent that can dissolve the porous ether cellulose derivative and a solvent that cannot dissolve the porous ether cellulose derivative after a step of causing the porous ether cellulose derivative microparticles to support a physiologically active substance to provide particles for preparation containing the physiologically active substance in the fine pores of the porous ether cellulose derivative microparticles; and a method of performing a step of bringing vapor of a solvent that can dissolve the porous ether cellulose derivative in contact after a step of causing the porous ether cellulose derivative microparticles to support a physiologically active substance to provide particles for preparation containing the physiologically active substance in the fine pores of the porous ether cellulose derivative microparticles. The types of the solvents and the mixing ratios in the mixed solvents in the above methods can be appropriately selected depending on the solubility of the ether cellulose derivative.

Furthermore, when modifying the surface of the porous ether cellulose derivative microparticles in the above methods, the degree of surface modification of the porous ether cellulose derivative microparticles can be controlled by controlling the type of each solvent and the mixing ratio of the mixed solvent or by controlling the time or amount of contact with each solvent or mixed solvent. The elution rate of the physiologically active substance supported on the porous ether cellulose derivative microparticles can be thus controlled.

Specifically, to control the mixing ratio between the solvent that can dissolve the ether cellulose derivative and the solvent that cannot dissolve the ether cellulose derivative, the proportion of the solvent that can dissolve the ether cellulose derivative in the mixed solvent is preferably 10 to 90 vol%, further preferably 20 to 70 vol%, even more preferably 30 to 50 vol%.

In a preferable aspect, the porous ether cellulose derivative microparticles used in the embodiment of the present invention exhibit particularly good fluidity when mixed with another additive such as an excipient. If the fluidity is poor, the amount to be filled becomes nonuniform in a filling step of forming capsule preparations or tablets, which results in decrease in uniformity in the preparations. Since the particles for preparation according to the embodiment of the present invention have high sphericity and good fluidity, the particles not only exhibit good uniformity in the preparations but also enhance efficiency of impartation of functionality by preventing the particles from fusing and/or adhering to each other, which occurs in the case where particles with low sphericity are used when applying a functional coating, to prevent generation of secondary particles and prevent layering of the sustained-release layer from becoming nonuniform.

In the case where the preparation obtained in the embodiment of the present invention is a tablet or an orally disintegrating tablet, the porous ether cellulose derivative microparticles used for the particles for preparation desirably retain their shape without being fractured by compression. It is preferable that the particles for preparation according to the embodiment of the present invention include porous microparticles having an ether cellulose derivative content of equal to or more than 50 mass% because such particles can retain their shape without experiencing brittle fracture compared with common inorganic porous particles.

The preparation according to the embodiment of the present invention may contain as appropriate additives such as pharmaceutically acceptable sugars or sugar alcohols (such as D-mannitol, lactose, erythritol, trehalose, xylitol, maltitol, sorbitol, and sucrose), disintegrators, inorganic excipients (such as anhydrous calcium hydrogen phosphate), starches, and other various additives used in production of common preparations. Examples of such additives include excipients, lubricants, binding agents, plasticizers, corrigents, perfumes, coloring agents, sweetenings, and surfactants.

Examples of the lubricants include magnesium stearate, calcium stearate, sodium stearyl fumarate, talc, sucrose fatty acid esters, stearic acid, aluminum stearate, potassium sodium tartrate, light anhydrous silicic acid, carnauba wax, carmellose calcium, carmellose sodium, hydrated silicon dioxide, hardened oils, and hardened rapeseed oil.

Examples of the binding agents include gelatin; pullulan; carrageenan; xanthan gum; tamarind gum; pectin; sodium alginate; water-soluble polysaccharides such as gum arabic; celluloses such as hydroxypropyl cellulose, hydroxypropyl methyl cellulose, and methyl cellulose; starches such as $\alpha$-starch and starch paste; and synthetic polymers such as polyvinylpyrrolidone, carboxyvinyl polymers, and poly(vinyl alcohol).

Examples of the plasticizers include talc, hydrated silicon dioxide, and light anhydrous silicic acid.

Examples of the disintegrators include crospovidone, sodium croscarmellose, calcium carmellose, sodium carboxymethyl starch, and low-substituted hydroxypropyl cellulose.

Examples of the corrigents include glutamic acid, fumaric acid, succinic acid, citric acid, sodium citrate, tartaric acid, malic acid, ascorbic acid, sodium chloride, and menthol.

Examples of the perfumes include orange-, vanilla-, strawberry-, and yogurt-flavored perfumes, menthol, and the above-described perfumes.

Examples of the coloring agents include titanium oxide, iron sesquioxide, yellow iron sesquioxide, talc, food colors such as Food Red No.3, Food Yellow No. 5, and Food Blue No. 1, and riboflavin.

Examples of the sweetenings include aspartame, saccharin, dipotassium glycyrrhizinate, stevia, and the other above-described sweeteners.

Examples of the surfactants include phospholipids, glycerin fatty acid esters, poly(ethylene glycol) fatty acid esters, sorbitan fatty acid esters, and polyoxyethylene hardened castor oil.

Also, the porous ether cellulose derivative microparticles according to the embodiment of the present invention are not limited to the above applications as a carrier, an adsorbent, a concentrating agent, a sustained-release base material of a physiologically-inactive active ingredient, and particles for preparation containing a physiologically active substance, and can be practically used in various applications.

Specific examples of the applications include materials to be subjected to processing typified by injection molding and microfabrication; electronic and electric material components and electronic product casing components obtained using the above materials; additives such as thickeners for processing and dimensional stabilizers; materials for paint films and coatings in the forms of dispersions, coating solutions, and paints; fluidity improvers, lubricants, abrasives, and thickeners as powders; slippage improvers, antiblocking agents, gloss adjusting agents, and frosted finish agents for plastic films and sheets; various modifiers such as light diffusion agents, surface hardness improvers, and tenacity improvers for plastic films, sheets, and lenses; various inks; additives in applications as gloss adjusting agents and frosted finish materials for toner; additives in applications as gloss adjusting agents and frosted finish materials for various paints; spacers for liquid crystal display equipment; fillers for chromatography; additives for cosmetics; deodorizers; collecting agents for pollutants such as heavy oil and oils; and gas adsorbents.

[Examples]

The present invention will be described below on the basis of examples. The present invention is not limited to these examples.

(Measurement of Average Surface Pore Size)

To determine the average surface pore size of microparticles in each example or the like described below, the surface of porous ether cellulose derivative microparticles was observed at magnifications of 10,000 to 50,000 times using a scanning electron microscope (FE-SEM, Scanning Electron Microscope JSM-6301NF manufactured by JEOL Ltd.) to measure the diameters (particle diameters) of 100 surface pore sizes. At that time, to determine an accurate average surface pore size reflecting the variation, observation was performed under such a magnification and field that equal to or more than ten surface pores were present in one image to measure the surface pore sizes for equal to or more than two porous ether cellulose derivative microparticles. Subsequently, the average surface pore size was calculated by determining the arithmetic mean of 100 surface pore sizes from the equation below. In the case where a surface pore was not perfect circular (for example, elliptic) in an image, its maximum diameter was measured as the pore size. In the case where fine pores communicated with one another to form an irregular shape, the maximum diameter of the connected pore was measured as the pore size.

[Math. 7]

$$[\text{Math.7}]$$

$$P = \left( \sum_{i=1}^{n} P_i \right) / n$$

(In the equation, $P_i$ represents the pore size of an individual surface pore, P represents the average surface pore size, and n represents the number of measurements, which was 100 in each example or the like described below.)

(Measurement of Linseed Oil Absorption)

The linseed oil absorption of the microparticles in each example or the like described below was measured according to the method described in Japan Industrial Standard (JIS) K 5101-13-1.

(Measurement of Pore Volume by Mercury Intrusion Method)

When determining the pore volume and the mode pore diameter of the microparticles in each example or the like described below, 0.1 g of a sample enclosed in a glass cell was subjected to measurement using a mercury porosimeter (AutoPore IV 9510 manufactured by Micrometrics Instrument Corporation) under the conditions below to provide an integrated pore size distribution curve and a differential pore size distribution curve. An integrated value of the volume derived from the fine pores excluding the volume derived from gaps between particles appearing in a region near the average particle diameter or equal to or larger than the average particle diameter was determined as the pore volume on the basis of the resulting integrated pore size distribution curve. Also, the pore size indicated by the peak top of the highest peak among peaks appearing not near the average particle diameter but near the average surface pore size was regarded as the mode pore diameter on the basis of the resulting differential pore size distribution curve.

<Measurement Conditions>

Measuring range of pore sizes: 0.004 to 400 $\mu$m
Contact angle of mercury: 141 degrees
Surface tension of mercury: 484 dyn/cm

(Measurement of Average Inter-Surface-Pore Distance)

The average inter-surface-pore distance of the microparticles in each example or the like described below is the arithmetic mean of values determined by measuring a distance from each of 30 surface pores randomly selected to an adjacent fine pore closest to the fine pore in the FE-SEM images used for measuring the average pore size. Specifically, calculation was carried out according to the equation below.

[Math. 8]

$$[\text{Math.8}]$$

$$L = \left( \sum_{i=1}^{n} L_i \right) / n$$

(In the equation, Li represents the shortest distance between an arbitrary fine pore and the closest adjacent fine pore, L represents the average inter-surface-pore distance, and n represents the number of measurements, which was 30 in each example or the like described below.)

(Measurement of Average Particle Diameter)

To determine the average particle diameter of the microparticles in each example or the like described below, the porous ether cellulose derivative microparticles were observed at magnifications of 100 to 500 times using an FE-SEM to measure the diameters (particle diameters) of 100 microparticles. At that time, to determine an accurate average particle diameter reflecting the variation of the particle diameters, observation was performed under such a magnification and field that 2 to less than 100 microparticles are present in an image to measure the particle diameters. Subsequently, the average particle diameter was calculated by determining the arithmetic mean of the particle diameters of the 100 microparticles from the equation below. In the case where a microparticle was not perfect circular (for example, elliptic) in an image, its maximum diameter was measured as the particle diameter. In the case where microparticles gathered to form an irregular aggregate, the diameter of a microparticle serving as the minimum unit constituting the aggregate was measured as the particle diameter. In the case where the aggregate was formed by fusion of a plurality of microparticles and showed no certain boundaries between the microparticles, the maximum diameter of the fused body was measured as the particle diameter.

[Math. 9]

$$[\text{Math.9}]$$

$$D = \left( \sum_{i=1}^{n} D_i \right) / n$$

(In the equation, Di represents the particle diameter of an individual microparticle, D represents the average particle diameter, and n represents the number of measurements, which was 100 in each example or the like described below.)

(Measurement of Sphericity)

The sphericity of the microparticles in each example or the like described below is the arithmetic mean of the sphericities of 30 microparticles randomly selected in the FE-SEM images used for determining the average particle diameter, and was calculated according to the equation below. The sphericity is the ratio between the major axis (maximum diameter) and the minor axis that intersects perpendicularly to the major axis at the center of the major axis of an individual microparticle, and was calculated according to the equation below.

[Math. 10]

## [Math.10]

$$S_m = \left( \sum_{i=1}^{n} S_i \right)/n$$

$$S_i = (a_i \,/\, b_i) * 100$$

(In the equation, Sm represents the average sphericity (%), Si represents the sphericity of an individual microparticle, ai represents the minor axis of the individual microparticle, bi represents the major axis of the individual microparticle, and n represents the number of measurements, which was 30 in each example or the like described below.)

(Measurement of Particle Diameter Distribution Index PDI)

The particle diameter distribution index PDI of the porous ether cellulose derivative microparticles was calculated from the equation below using the results of the measurements of the particle diameters of individual microparticles performed when calculating the average particle diameter.
[Math. 11]

## [Math.11]

$$PDI = \frac{\left( \sum_{i=1}^{n} D_i^{\,4} \right)/\left( \sum_{i=1}^{n} D_i^{\,3} \right)}{\left( \sum_{i=1}^{n} D_i \right)/n}$$

(In the equation, Di represents the particle diameter of an individual particle, PDI represents the particle diameter distribution index, and n represents the number of measurements, which was 100 in each example or the like described below.)

(Measurement of Bulk Density)

The bulk density of the microparticles in each example or the like described below was calculated according to the equation below through precisely weighing 4.00 g of the microparticles (the mass of the microparticles at this time is represented as W), then gently putting the weighed microparticles having a mass of W (g) into a 50-mL graduated cylinder (with a minimum scale of 1 mL), a 25-mL graduated cylinder (with a minimum scale of 0.5 mL), or a 10-mL graduated cylinder (with a minimum scale of 0.2 mL), and visually measuring the volume V of the powder layer.
[Math. 12]

## [Math.12]

$$\text{Bulk density} = \frac{W}{V}$$

(In the equation, W represents the mass of the microparticles, and V represents the volume of the powder layer measured with a graduated cylinder.)

(Measurement of Degree of Crystallinity)

To determine the degrees of crystallinity of the ether cellulose derivative and the porous ether cellulose derivative microparticles, X-ray diffraction patterns were measured with an X-ray powder diffractometer (RINT-2100 manufactured by Rigaku Corporation) using CuK$\alpha$ as the X-ray at diffraction angles of the X-ray of 5 to 70° and a scan rate of 2°/min with a sampling width of 0.02°. After that, the diffraction patterns were each subjected to waveform separation into the crystalline portion and the amorphous portion, and the degree of crystallinity = (the scattering intensity from the crystalline portion)/(the total scattering intensity) $\times$ 100 was calculated.

(Evaluation of Change in Fragrance over Time from Composite Microparticles Containing Perfume)

An arbitrary amount of composite microparticles containing a perfume was put on a petri dish, the petri dish was put on a hot plate at 35°C, and the change in fragrance over time from the composite microparticles was evaluated by giving scores as follows through sensory evaluation by 5 panelists (5: very strongly fragrant, 4: strongly fragrant, 3: moderately fragrant, 2: slightly fragrant, 1: almost no fragrance).

[Example 1]

To a 200-mL separable flask were added 5 parts by mass of ethyl cellulose ('Aqualon (registered trademark)' EC-N50 manufactured by Ashland Inc., ethoxy group content by percentage: 49.0 mass% (degree of substitution of the ethoxy group: 2.54), viscosity (80 mass% toluene/20 mass% ethanol solution, ethyl cellulose concentration: 5 mass%, 25°C): 47 m Pa·s, degree of crystallinity: 2.0%) as an ether cellulose derivative (A), 5 parts by mass of polyvinylpyrrolidone (K-85N manufactured by Nippon Shokubai Co., Ltd., weight-average molecular weight: 1,100,000) as a polymer (B), and 90 parts by mass of ethanol (manufactured by Amakasu Chemical Industries, extra pure) as an alcohol solvent (C). These raw materials were heated to 70°C in 15 minutes with stirring at a rotation rate of a stirring blade of 300 rpm and stirred for 2 hours at a rotation rate of 300 rpm while being held at 70°C. Subsequently, 200 parts by mass of deionized water was added dropwise as a poor solvent (D) through a feeding pump at a rate of 1.7 parts by mass/min with stirring at 300 rpm to provide a suspension. The resulting suspension was subjected to solid-liquid separation by filtration under reduced pressure and washed with 100 parts by mass of deionized water, and the separated solid matter was vacuum-dried at 50°C to provide ethyl cellulose microparticles.

Observation of the resulting ethyl cellulose microparticles under an FE-SEM showed that the surface was porous. The average surface pore size was 0.68 pm, the average inter-surface-pore distance was 0.49 pm (0.72 times as large as the average surface pore size), the linseed oil absorption was 151 mL/100 g, the average particle diameter was 44.7 pm, the sphericity was 94.4%, the PDI was 1.32, the bulk density was 0.28 g/mL, and the degree of crystallinity of the microparticles was 3.8%.

[Example 2]

The raw materials in Example 1 were changed as follows. That is, 9 parts by mass of ethyl cellulose ('Aqualon (registered trademark)' EC-N50 Pharm manufactured by Ashland Inc., ethoxy group content by percentage: 49.0 mass% (degree of substitution of the ethoxy group: 2.54), viscosity (80 mass% toluene/20 mass% ethanol solution, ethyl cellulose concentration: 5 mass%, 25°C): 45 m Pa·s, degree of crystallinity: 13%) was used as the ether cellulose derivative (A), 8 parts by mass of polyvinylpyrrolidone (K-30 manufactured by Wako Pure Chemical Industries, Ltd., weight-average molecular weight: 50,000) was used as the polymer (B), 83 parts by mass of ethanol (manufactured by Amakasu Chemical Industries, extra pure) was used as the alcohol solvent (C), and 140 parts by mass of deionized water was used as the poor solvent (D). Formation of microparticles was carried out through substantially the same operations as in Example 1 except that the rate of dropping the poor solvent (D) through the feeding pump was 2.8 parts by mass/min.

Observation of the resulting ethyl cellulose microparticles under an FE-SEM showed that the surface was porous. The average surface pore size was 1.4 $\mu$m, the average inter-surface-pore distance was 1.2 pm (0.86 times as large as the average surface pore size), the linseed oil absorption was 102 mL/100 g, the average particle diameter was 213 pm, the sphericity was 91.9%, the PDI was 1.13, the bulk density was 0.38 g/mL, and the degree of crystallinity of the microparticles was 3.1%.

[Example 3]

The raw materials in Example 1 were changed as follows. That is, 5 parts by mass of ethyl cellulose ('Aqualon (registered trademark)' EC-N50 Pharm manufactured by Ashland Inc., ethoxy group content by percentage: 49.0 mass% (degree of substitution of the ethoxy group: 2.54), viscosity (80 mass% toluene/20 mass% ethanol solution, ethyl cellulose concentration: 5 mass%, 25°C): 45 m Pa·s, degree of crystallinity: 13%) was used as the ether cellulose derivative (A), 10 parts by mass of polyvinylpyrrolidone (K-30 manufactured by Wako Pure Chemical Industries, Ltd., weight-average molecular weight: 50,000) was used as the polymer (B), 85 parts by mass of ethanol (manufactured by Amakasu Chemical Industries, extra pure) was used as the alcohol solvent (C), and 140 parts by mass of deionized water was used as the poor solvent (D). Formation of microparticles was carried out through substantially the same operations as in Example 1 except that the rate of dropping the poor solvent (D) through the feeding pump was 2.8 parts by mass/min.

Observation of the resulting ethyl cellulose microparticles under an FE-SEM showed that the surface was porous. The average surface pore size was 0.79 pm, the average inter-surface-pore distance was 0.75 pm (0.95 times as large as the average surface pore size), the linseed oil absorption was 100 mL/100 g, the average particle diameter was 122 pm, the sphericity was 99.0%, the PDI was 1.19, the bulk density was 0.46 g/mL, and the degree of crystallinity of the microparticles was 2.9%.

[Example 4]

The raw materials in Example 1 were changed as follows. That is, 5 parts by mass of ethyl cellulose ('Aqualon (registered trademark)' EC-T50 manufactured by Ashland Inc., ethoxy group content by percentage: 50.3 mass% (degree of substitution of the ethoxy group: 2.64), viscosity (80 mass% toluene/20 mass% ethanol solution, ethyl cellulose concentration: 5 mass%, 25°C): 41 m Pa-s, degree of crystallinity: 18%) was used as the ether cellulose derivative (A), 5 parts by mass of polyvinylpyrrolidone (K-85N manufactured by Nippon Shokubai Co., Ltd., weight-average molecular weight: 1,100,000) was used as the polymer (B), and 90 parts by mass of ethanol (manufactured by Amakasu Chemical Industries, extra pure) was used as the alcohol solvent (C). In Example 4, formation of microparticles was carried out through substantially the same operations as in Example 1.

Observation of the resulting ethyl cellulose microparticles under an FE-SEM showed that the surface was porous. The average surface pore size was 0.47 $\mu$m, the average inter-surface-pore distance was 0.16 $\mu$m (0.34 times as large as the average surface pore size), the linseed oil absorption was 466 mL/100 g, the average particle diameter was 55.4 $\mu$m, the sphericity was 78.5%, the PDI was 1.31, the bulk density was 0.11 g/mL, and the degree of crystallinity of the microparticles was 4.2%.

[Example 5]

The raw materials in Example 1 were changed as follows. That is, 7 parts by mass of ethyl cellulose ('Aqualon (registered trademark)' EC-N50 manufactured by Ashland Inc., ethoxy group content by percentage: 49.0 mass% (degree of substitution of the ethoxy group: 2.54), viscosity (80 mass% toluene/20 mass% ethanol solution, ethyl cellulose concentration: 5 mass%, 25°C): 47 m Pa·s, degree of crystallinity: 2.0%) was used as the ether cellulose derivative (A), 10 parts by mass of polyvinylpyrrolidone (K-30 manufactured by Wako Pure Chemical Industries, Ltd., weight-average molecular weight: 50,000) was used as the polymer (B), 83 parts by mass of ethanol (manufactured by Amakasu Chemical Industries, extra pure) was used as the alcohol solvent (C), and 140 parts by mass of deionized water was used as the poor solvent (D). Formation of microparticles was carried out through substantially the same operations as in Example 1 except that the rate of dropping the poor solvent (D) through the feeding pump was 1.0 parts by mass/min.

Observation of the resulting ethyl cellulose microparticles under an FE-SEM showed that the surface was porous. The average surface pore size was 1.3 $\mu$m, the average inter-surface-pore distance was 1.5 pm (1.2 times as large as the average surface pore size), the linseed oil absorption was 73 mL/100 g, the average particle diameter was 152 pm, the sphericity was 75.9%, the PDI was 3.10, the bulk density was 0.41 g/mL, and the degree of crystallinity of the microparticles was 1.7%.

[Example 6]

In a 10-L autoclave were put 7 parts by mass of ethyl cellulose ('Aqualon (registered trademark)' EC-N50 Pharm manufactured by Ashland Inc., ethoxy group content by percentage: 49.0 mass% (degree of substitution of the ethoxy group: 2.54), viscosity (80 mass% toluene/20 mass% ethanol solution, ethyl cellulose concentration: 5 mass%, 25°C): 45 m Pa·s, degree of crystallinity: 13%) as the ether cellulose derivative (A), 10 parts by mass of polyvinylpyrrolidone (K-30 manufactured by Wako Pure Chemical Industries, Ltd., weight-average molecular weight: 50,000) as the polymer (B), and 83 parts by mass of ethanol (manufactured by Amakasu Chemical Industries, extra pure) as the alcohol solvent (C). These raw materials were heated to 70°C in 60 minutes with stirring at a rotation rate of a stirring blade of 170 rpm and stirred for 2 hours at a rotation rate of 170 rpm while being held at 70°C. Subsequently, 125 parts by mass of deionized water was added dropwise as the poor solvent (D) through a feeding pump at a rate of 2.8 parts by mass/min with stirring at 170 rpm to provide a suspension. The resulting suspension was subjected to solid-liquid separation by filtration under reduced pressure and washed with 100 parts by mass of deionized water, and the separated solid matter was vacuum-dried at 50°C to provide ethyl cellulose microparticles.

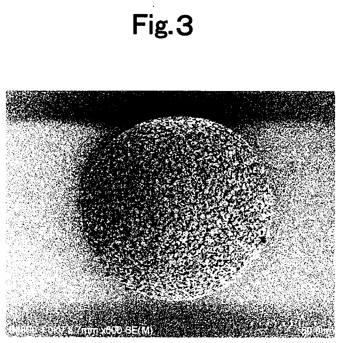

Observation of the resulting ethyl cellulose microparticles under an FE-SEM (at a magnification of 600 times) showed that the surface was porous. The average surface pore size was 0.63 pm, the average inter-surface-pore distance was 0.57 pm (0.90 times as large as the average surface pore size), the linseed oil absorption was 103 mL/100 g, the average particle diameter was 101 pm, the sphericity was 98.7%, the PDI was 1.10, the bulk density was 0.39 g/mL, and the degree of crystallinity of the microparticles was 3.0%. The pore volume calculated by the mercury intrusion method was 0.41 cm$^3$/g, and the mode pore diameter was 0.65 pm (1.01 times as large as the average surface pore size). Fig. 3 shows an image of the resulting ethyl cellulose microparticle observed under an FE-SEM at a magnification of 600 times.

[Comparative Example 1]

The raw materials in Example 1 were changed as follows. That is, 5 parts by mass of ethyl cellulose ('Aqualon (registered trademark)' EC-K50 manufactured by Ashland Inc., ethoxy group content by percentage: 45.8 mass% (degree of substitution of the ethoxy group: 2.29), viscosity (80 mass% toluene/20 mass% ethanol solution, ethyl cellulose concentration: 5 mass%, 25°C): 42 m Pa·s) was used as the ether cellulose derivative (A), 5 parts by mass of polyvinylpyrrolidone (K-85N manufactured by Nippon Shokubai Co., Ltd., weight-average molecular weight: 1,100,000) was used as the polymer (B), and 90 parts by mass of ethanol (manufactured by Amakasu Chemical Industries, extra pure) was used as the alcohol solvent (C). In Comparative Example 1, formation of microparticles was carried out through substantially the same operations as in Example 1.

Evaluation of the degree of crystallinity of 'Aqualon (registered trademark)' EC-K50 used in Comparative Example 1 by X-ray powder diffraction showed no detectable main peak attributable to the crystalline portion of ethyl cellulose, which indicated that the polymer was amorphous.

The resulting ethyl cellulose microparticle observed under an FE-SEM was an ethyl cellulose microparticle (nonporous ethyl cellulose microparticle) having a smooth surface with no fine pores observed on the surface of the microparticle. The linseed oil absorption of the resulting ethyl cellulose microparticles was 32 mL/100 g, the average particle diameter was 31.2 $\mu$m, and the pore volume calculated by the mercury intrusion method was 0.015 cm$^3$/g. Like the X-ray diffraction pattern of ethyl cellulose used as a raw material, the X-ray diffraction pattern of the resulting ethyl cellulose microparticles showed no peak attributable to the crystalline portion of ethyl cellulose, which indicated that the microparticles were amorphous.

Table 1 below shows the results of measurement on Examples 1 to 6 and Comparative Example 1.

[Table 1]

[Table1]

|  | Degree of crystallinity of raw material(%) | Degree of crystallinity of particles(%) | Average particle diameter D ($\mu$m) | Linseed oil absorption (mL/100g) | Average surface pore size P ($\mu$m) | Sphericity Sm (%) | Particle diameter distribution index PDI | Bulk density (g/mL) |
|---|---|---|---|---|---|---|---|---|
| Example 1 | 2.0 | 3.8 | 44.7 | 151 | 0.68 | 94.4 | 1.32 | 0.28 |
| Example 2 | 13 | 3.1 | 213 | 102 | 1.4 | 91.9 | 1.13 | 0.38 |
| Example 3 | 13 | 2.9 | 122 | 100 | 0.79 | 99.0 | 1.19 | 0.46 |
| Example 4 | 18 | 4.2 | 55.4 | 466 | 0.47 | 78.5 | 1.31 | 0.11 |
| Example 5 | 2.0 | 1.7 | 152 | 73 | 1.3 | 75.9 | 3.10 | 0.41 |
| Example 6 | 13 | 3.0 | 101 | 103 | 0.63 | 98.7 | 1.10 | 0.39 |
| Comparative Example 1 | (amorphous) | (amorphous) | 26.4 | 32 | (Nonporous) |  |  |  |

[Comparative Example 2]

The pore volume of powder of ethyl cellulose ('Aqualon (registered trademark)' EC-N50 Pharm manufactured by Ashland Inc., ethoxy group content by percentage: 49.0 mass% (degree of substitution of the ethoxy group: 2.54)) used as a raw material in Examples 2, 3, and 6 was measured by the mercury intrusion method. The pore volume calculated was 0.02 cm$^3$/g, and the powder of ethyl cellulose used as a raw material did not have a porous structure.

[Example 7]

Use of the porous ethyl cellulose microparticles obtained in Example 1 as a silicone-oil collecting agent was considered. To water in which 5 mL of silicone oil (SRX 310 manufactured by Dow Corning Toray Co., Ltd.) was floating, 5g of the porous ethyl cellulose microparticles in Example 1 was added. It was observed that the porous ethyl cellulose microparticles in Example 1 selectively gathered around silicone oil droplets floating in water. After that, the porous cellulose microparticles floating in water were collected, and the silicone oil droplets that had been floating in water disappeared. Subsequently, the porous ethyl cellulose microparticles collected was added to 50 g of ethanol and stirred at 50°C. The porous ethyl cellulose was dissolved in ethanol, and it was observed that silicone oil droplets had sunk in ethanol. It was confirmed that the porous ethyl cellulose selectively collected the oil droplets in water and that oil droplets could be collected from the porous ethyl cellulose microparticles that had absorbed the oil.

[Example 8]

To 2,955 parts by mass of deionized water, 45 parts by mass of vanillin (manufactured by Wako Pure Chemical Industries, Ltd.) was dissolved at 40°C to provide 3,000 parts by mass of a 1.5 mass% aqueous solution of vanillin. Subsequently, 100 parts by mass of the porous ethyl cellulose microparticles obtained in Example 6 was put on a piece of filter paper No. 5C (with a diameter of 21 mm) (manufactured by Kiriyama Glass Works Co.) for Kiriyama Rhoto funnels placed on a Kiriyama Rhoto funnel (manufactured by Kiriyama Glass Works Co.), and 3,000 parts by mass of the 1.5 mass% aqueous solution of vanillin was poured on the Kiriyama Rhoto funnel while performing suction filtration. After suction filtration of the aqueous solution of vanillin, the microparticles on the filter paper was vacuum-dried at 50°C to provide a powder of white microparticles that exhibited fragrance of vanillin. Observation of the resulting powder of the microparticles at magnifications of 500, 1,500, and 3,000 times under an FE-SEM showed that the surface of the microparticles was porous. Also, it did not appear that the outer surface of the microparticles supported coarse vanillin crystals or that independent coarse vanillin crystals were mixed with the microparticles, which indicated that composite microparticles containing vanillin in the fine pores of the porous ethyl cellulose microparticles were obtained.

[Comparative Example 3]

Supporting of vanillin was carried out through substantially the same operations as in Example 8 except that the nonporous ethyl cellulose microparticles in Comparative Example 1 were used instead of the porous ethyl cellulose microparticles obtained in Example 6, so that a powder of white microparticles that exhibited fragrance of vanillin was obtained. Observation of the resulting powder of the microparticles at 500, 2,000, and 5,000 times under an FE-SEM showed that crude crystals of vanillin adhered to the surface of the microparticles, which indicated that vanillin and the ethyl cellulose microparticles were mixed to form the powder.

[Test Example 1; Evaluation of Sustained-Release Properties for Particles Obtained in Example 8 and Comparative Example 3]

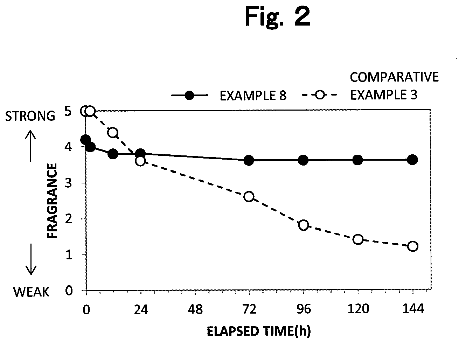

The same mass of the composite microparticles of vanillin and the porous ethyl cellulose obtained in Example 8 and the mixed powder of vanillin and the nonporous ethyl cellulose obtained in Comparative Example 3 were put on different petri dishes and put on a hot plate at 35°C, and the change in fragrance over time from each sample was evaluated by giving scores through sensory evaluation by 5 panelists. Fig. 2 shows the change over time of the average scores of fragrance strengths given through sensory evaluation by the five panelists. The composite microparticles of vanillin and the porous ethyl cellulose obtained in Example 8 exhibited better sustained-release performance of a perfume than the sustained-release performance of the mixed powder of vanillin and the nonporous ethyl cellulose obtained in Comparative Example 3.

[Test Example 2; Measurement of Water Vapor Adsorption Isotherm]

The water vapor adsorption isotherm of each of the porous ethyl cellulose microparticles obtained in Example 6, porous calcium silicate ("FLORITE (registered trademark)" R manufactured by Tomita Pharmaceutical Co., Ltd.), and silica gel ("SYLOID (registered trademark)" 3150 manufactured by W. R. Grace & Co.) was measured under the following conditions.

Apparatus: VTI-SA+ (manufactured by TA Instruments, Inc.)
Drying temperature: 60°C
Measurement temperature: 25°C
Maximum balancing time: 60 minutes
Equilibrium criterion: 0.01 wt%/5 min
RH step: 5 to 95% RH (every 5% RH)

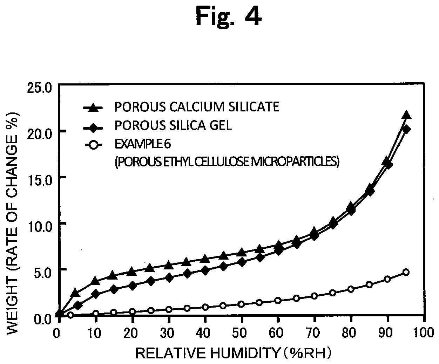

Fig. 4 shows the results. As shown in Fig. 4, the microparticles in Example 6 exhibited lower hygroscopicity than the hygroscopicity of the existing inorganic porous particles. Accordingly, it was confirmed that the microparticles in Example 6 could stably store a physiologically active substance that becomes unstable due to external water.

[Test Example 3; Measurement of Particle Strength]

The particle strengths of the porous ethyl cellulose microparticles obtained in Example 6 and silica gel were evaluated by the following method. The load-compression curve when a load was applied to the microparticles was derived using a micro compression tester (MCT-210 manufactured by Shimadzu Corporation), and the strength against the load was evaluated on the basis of the resulting load-compression curve. Specifically, the microparticles were disposed on a platen of the micro compression tester, the diameter D of a microparticle randomly selected from the microparticles was measured, and a displacement L of the microparticle when loads of up to 100 mN were applied with a diamond indenter with a diameter of 50 $\mu$m under a condition of a compression rate of 13.3240 mN/sec was measured. A load-compression curve was made using the obtained displacement L with the horizontal axis indicating the compressibility C (%; C = L/D $\times$ 100) of the microparticles and the vertical axis indicating the load F (mN), and evaluations were made on the basis of the shape of the curve to determine whether the microparticles exhibited displacement along with the load or experienced brittle fracture due to the load. The measurement involving changes in the load was carried out six times for each type of the microparticles. The load-compression curve was made for each measurement to evaluate the strength of the microparticles against the load.

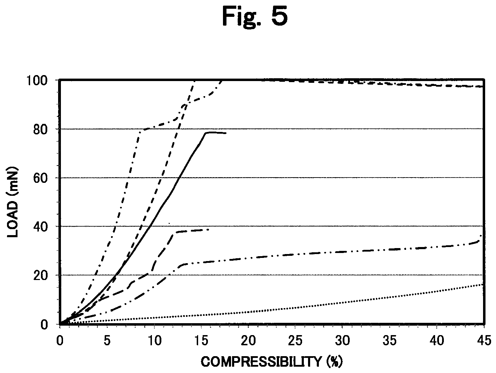

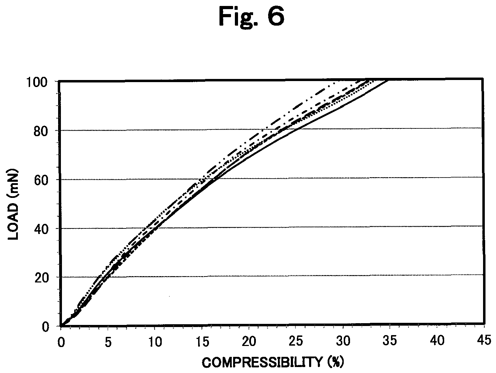

Fig. 5 and Fig. 6 show the results. Fig. 5 shows the results for the silica gel particles, and Fig. 6 shows the results for the porous ethyl cellulose microparticles in Example 6. In each of Fig. 5 and Fig. 6, the load-compression curves based on the six respective measurements are shown together. Although silica gel, which is existing inorganic porous particles, experienced brittle fracture as shown in Fig. 5, the microparticles in Example 6 were elastic under the compressive stress as shown in Fig. 6. The above results showed that particles are less likely to be damaged in the production process in the case where composite microparticles containing an active ingredient are produced using the microparticles in Example 6 or in the case where a preparation containing such composite microparticles is produced. Hence, it is deemed possible that the sustained-release dissolution profile of the porous ethyl cellulose microparticles is retained even when forming the composite microparticles into a tablet.

[Test Example 4; Measurement of Sphericity]

Measurement of the sphericity was carried out on each of a powder of ethyl cellulose ('Aqualon (registered trademark)' EC-N50 Pharm manufactured by Ashland Inc.), which was used as the raw material ethyl cellulose in Example 6 and was not porous, the porous ethyl cellulose microparticles in Example 6, silica gel ("SYLOID (registered trademark)" 3150 manufactured by W. R. Grace & Co.), and porous calcium silicate ("FLORITE (registered trademark)" R manufactured by Tomita Pharmaceutical Co., Ltd.).

[Table 2]

[Table2]

|  | Ethyl cellulose | Porous EC particles in Example 6 | Silica gel | Porous calcium silicate |
|---|---|---|---|---|
| Sphericity | 67.1 | 98.7 | 75.2 | 75.9 |

As shown in Table 2, the sphericity of the microparticles in Example 6 was extremely high.

[Test Example 5; Interaction with Physiologically Active Substance]

Using a 200-mL volumetric flask, 20 mg of quinine hydrochloride dihydrate (manufactured by Wako Pure Chemical Industries, Ltd., extra pure) was diluted with deionized water to provide a 0.1 mg/mL aqueous solution of quinine hydrochloride dihydrate. Using a 200-mL volumetric flask, 20 mg of thiamine hydrochloride (manufactured by Acros Organics N.V.) was dissolved in deionized water to provide a 0.1 mg/mL aqueous solution of thiamine hydrochloride. To 10-mL glass tubes, 5 mL each of the two aqueous solutions of the physiologically active substances were respectively transferred and regarded as undiluted solutions. To each undiluted solution, 0.5 g of the porous ethyl cellulose microparticles in Example 6 or silica gel ("SYLOID (registered trademark)" 3150 manufactured by W. R. Grace & Co.) was added, and the mixture was stirred for 1 minute with a vortex mixer (MT-31 manufactured by Yamato Scientific Co., Ltd.) and then allowed to stand still for a night. Each solution after being allowed to stand still was passed through a 0.45-$\mu$m filter to provide an HPLC sample.

<HPLC Conditions>

Mobile phase A: 20 mM aqueous solution of potassium dihydrogenphosphate/acetonitrile = 95/5 (v/v)
Mobile phase B: 20 mM aqueous solution of potassium dihydrogenphosphate/acetonitrile = 40/60 (v/v)
Column: "Capcell Pak (registered trademark)" MGII (manufactured by Shiseido Company, Ltd., 3.0 × 150 mm)
Column temperature: 40°C
Detection wavelength: 280 nm
Injection volume: 10 $\mu$L
Gradient: 0 minutes ··· mobile phase A: 100%
10 minutes ··· mobile phase A: 100%
25 minutes ··· mobile phase B: 100%
30 minutes ··· mobile phase A: 100%

Table 3 shows the residual ratios of the physiologically active substances in the solutions determined by HPLC. The residual ratios (%) were calculated from the equation below.
[Math. 13]

[Math.13]

$$\text{Residual ratio} = \frac{\text{HPLC peak area of physiologically active substance in sample solution}}{\text{HPLC peak area of physiologically active substance in undiluted solution}}$$

[Table 3]

[Table3]

| | Undiluted solution | Example 6 | | Silica gel | |
|---|---|---|---|---|---|
| | HPLC peak area | HPLC peak area | Residual ratio (%) | HPLC peak area | Residual ratio (%) |
| Quinine hydrochloride dihydrate | 2,904,700 | 2,503,118 | 86 | 768,910 | 26 |
| Thiamine hydrochloride | 9,002,763 | 9,833,589 | 109 | 2,602,011 | 29 |

As shown in Table 3, in the case where silica gel was used, the concentrations of the physiologically active substances in the solutions dropped significantly to provide residual ratios of equal to or less than 30%. Hence, silica gel is deemed to exhibit comparatively strong interaction with quinine hydrochloride dihydrate and thiamine hydrochloride, which are physiologically active substances. On the other hand, in the case where the microparticles in Example 6 were used, the drops in the concentrations of the physiologically active substances were suppressed, and the residual ratios were equal to or more than 85%. Hence, it was shown that the microparticles in Example 6 exhibited weak interaction with the physiologically active substances.

[Test Example 6; Linseed Oil Absorption]

The microparticles in Example 6 or crystalline cellulose ("Celphere (registered trademark)" CP102 manufactured by Asahi Kasei Corporation) was mixed with linseed oil (manufactured by Kanto Chemical Co., Inc.) little by little with a spatula. The linseed oil absorption (volume of the linseed oil consumed per 100 g of the sample) when the paste got a smooth hardness was calculated.

[Table 4]

[Table4]

|  | Example 6 | Crystalline cellulose |
|---|---|---|
| Linseed oil absorption (mL/100g) | 103 | 46 |

As shown in Table 4, the linseed oil absorption of the microparticles in Example 6 was larger than the linseed oil absorption of the existing crystalline cellulose core particles. Hence, the microparticles in Example 6 are deemed to be able to support a larger amount of physiologically active substances.

[Example 9]

Using a 50-mL volumetric flask, 25 g of sodium salicylate (manufactured by Kanto Chemical Co., Inc., guaranteed reagent) was dissolved in deionized water to provide a 0.5 g/mL aqueous solution of sodium salicylate. To a piece of filter paper, 10 g of the microparticles in Example 6 were transferred, 18 mL of the 0.5 g/mL aqueous solution of sodium salicylate was added, and suction filtration was carried out. Another 18 mL of the 0.5 g/mL aqueous solution of sodium salicylate was added, and suction filtration was carried out. The solid matter on the filter paper was dried at 50°C for 4 hours in a vacuum dryer to provide particles for preparation in which the porous ethyl cellulose microparticles supported sodium salicylate.

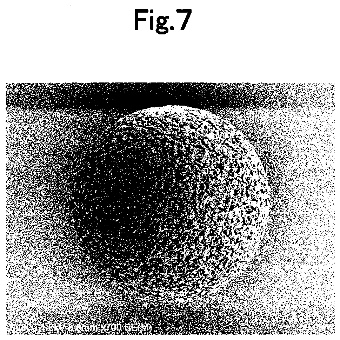

Fig. 7 shows an FE-SEM image (at a magnification of 700 times) of the particle for preparation in Example 9. Observation under an FE-SEM at magnifications of 700, 1,500, and 3,000 times showed that sodium salicylate was not mixed with the microparticles as independent crude crystals separated from the microparticles but was supported in the fine pores and on the surface of the microparticles.

[Comparative Example 4]

Ethyl cellulose particles supporting sodium salicylate were obtained in substantially the same manner as in Example 9 except that ethyl cellulose particles on the market ("ETHOCEL (registered trademark)" 100 Premium manufactured by The Dow Chemical Company) that were not porous were used instead of the microparticles in Example 6.

[Comparative Example 5]

Ethyl cellulose particles supporting sodium salicylate were obtained in substantially the same manner as in Example 9 except that ethyl cellulose particles on the market ("ETHOCEL (registered trademark)" 10 Premium manufactured by The Dow Chemical Company) that were not porous were used instead of the microparticles in Example 6.

[Test Example 7; Measurement of Fluidity of Particles]

To evaluate the fluidity of particles, measurement of the angle of repose was carried out on each of ethyl cellulose ('Aqualon (registered trademark)' EC-N50 Pharm manufactured by Ashland Inc.), which was the raw material, the microparticles in Example 6, silica gel ("SYLOID (registered trademark)" 3150), and porous calcium silicate ("FLORITE (registered trademark)" R manufactured by Tomita Pharmaceutical Co., Ltd.). Each sample was piled on a table for measurement using Powder Tester (PT-N manufactured by Hosokawa Micron Corporation), and the angle between the horizontal surface and the lateral surface of the conical pile was measured as the angle of repose of the sample. In the same manner, the angles of repose of the particles for preparation in Example 9 and the ethyl cellulose particles in Comparative Examples 4 and 5 were measured.

[Table 5]

[Table5]

|  | Ethyl cellulose | Example 6 | Silica gel | Porous Calcium silicate |
|---|---|---|---|---|
| Angle of repose (°) | 35.3 | 21.9 | 29.8 | 36.3 |

[Table 6]

[Table6]

|  | Example 9 | Comparative Example 4 | Comparative Example 5 |
|---|---|---|---|
| Angle of Repose (°) | 25.2 | 36.1 | 46.4 |

The results in Table 5 show that the porous ethyl cellulose microparticles in Example 6 had an angle of repose smaller than the angles of repose of the nonporous ethyl cellulose particles and the existing inorganic porous particles and had extremely good fluidity. The results in Table 6 show that the particles for preparation supporting the physiologically active substance in Example 9 also had high fluidity and are particles suitable for continuous production of preparations containing a physiologically active substance.

[Example 10]

A 50 vol% aqueous solution of ethanol was obtained by mixing 100 mL of deionized water and 100 mL of ethanol. Using a 10-mL volumetric flask, 230 mg of salicylic acid (manufactured by Kanto Chemical Co., Inc., extra pure) was dissolved in the 50 vol% aqueous solution of ethanol to provide a 23 mg/mL salicylic acid solution. Into an ultrafiltration membrane unit ("Nanosep (registered trademark)" 100K manufactured by Nihon Pall Ltd.), 30 mg of the microparticles in Example 6 were charged, 500 $\mu$L of the salicylic acid solution was added, and the mixture was subjected to centrifugal filtration (10,600 G, 5 minutes) using a centrifuge (Refrigerated Centrifuge 5417 R manufactured by Eppendorf AG). The solid matter on the filter was dried at 25°C for 16 hours in a vacuum dryer to provide particles for preparation in which the porous ethyl cellulose microparticles supported salicylic acid.

[Example 11]

Using a 10-mL volumetric flask, 70 mg of theophylline (manufactured by Kanto Chemical Co., Inc., guaranteed reagent) was dissolved in the 50 vol% aqueous solution of ethanol to provide a 7 mg/mL theophylline solution. Particles for preparation in which the porous ethyl cellulose microparticles supported theophylline were obtained in substantially the same manner as in Example 10.

[Example 12]

Using a 10-mL volumetric flask, 2.2 g of quinine hydrochloride dihydrate (manufactured by Wako Pure Chemical Industries, Ltd., extra pure) was dissolved in the 50 vol% aqueous solution of ethanol to provide a 220 mg/mL quinine hydrochloride dihydrate solution. Particles for preparation in which the porous ethyl cellulose microparticles supported quinine hydrochloride dihydrate were obtained in substantially the same manner as in Example 10.

[Example 13]

Using a 10-mL volumetric flask, 480 mg of thiamine hydrochloride (manufactured by Acros Organics N.V.) was dissolved in the 50 vol% aqueous solution of ethanol to provide a 48 mg/mL thiamine hydrochloride solution. Particles for preparation in which the porous ethyl cellulose microparticles supported thiamine hydrochloride were obtained in substantially the same manner as in Example 10.

[Example 14]

Using a 10-mL volumetric flask, 200 mg of acetaminophen (manufactured by Kanto Chemical Co., Inc.) was dissolved in the 50 vol% aqueous solution of ethanol to provide a 20 mg/mL acetaminophen solution. Particles for preparation in which the porous ethyl cellulose microparticles supported acetaminophen were obtained in substantially the same manner as in Example 10.

[Example 15]

Using a 10-mL volumetric flask, 50 mg of ibuprofen (manufactured by Acros Organics N.V.) was dissolved in the 50 vol% aqueous solution of ethanol to provide a 5 mg/mL ibuprofen solution. Particles for preparation in which the porous ethyl cellulose microparticles supported ibuprofen were obtained in substantially the same manner as in Example 10.

[Comparative Example 6]

Ethyl cellulose particles supporting salicylic acid were obtained in substantially the same manner as in Example 10 except that an ethyl cellulose powder ('Aqualon (registered trademark)' EC-N50 Pharm manufactured by Ashland Inc.) that was not porous and was used as the raw material was used instead of the microparticles in Example 6.

[Comparative Example 7]

Ethyl cellulose particles supporting theophylline were obtained in substantially the same manner as in Example 11 except that an ethyl cellulose powder ('Aqualon (registered trademark)' EC-N50 Pharm manufactured by Ashland Inc.) that was not porous and was used as the raw material was used instead of the microparticles in Example 6.

[Comparative Example 8]

Ethyl cellulose particles supporting quinine hydrochloride dihydrate were obtained in substantially the same manner as in Example 12 except that an ethyl cellulose powder ('Aqualon (registered trademark)' EC-N50 Pharm manufactured by Ashland Inc.) that was not porous and was used as the raw material was used instead of the microparticles in Example 6.

[Comparative Example 9]

Ethyl cellulose particles supporting thiamine hydrochloride were obtained in substantially the same manner as in Example 13 except that an ethyl cellulose powder ('Aqualon (registered trademark)' EC-N50 Pharm manufactured by Ashland Inc.) that was not porous and was used as the raw material was used instead of the microparticles in Example 6.

[Comparative Example 10]

Ethyl cellulose particles supporting acetaminophen were obtained in substantially the same manner as in Example 14 except that an ethyl cellulose powder ('Aqualon (registered trademark)' EC-N50 Pharm manufactured by Ashland Inc.) that was not porous and was used as the raw material was used instead of the microparticles in Example 6.

[Comparative Example 11]

Ethyl cellulose particles supporting ibuprofen were obtained in substantially the same manner as in Example 15 except that an ethyl cellulose powder ('Aqualon (registered trademark)' EC-N50 Pharm manufactured by Ashland Inc.) that was not porous and was used as the raw material was used instead of the microparticles in Example 6.

[Test Example 8; Comparison of Supported Amounts]

Measurement of the supported amounts of physiologically active substances was carried out by HPLC on the porous ethyl cellulose microparticles in Example 6 supporting the physiologically active substances (Examples 10 to 15) and the raw material ethyl cellulose powders supporting the same physiologically active substances (Comparative Examples 6 to 11). The supported amount ratio of a physiologically active substance between microparticles in an example and microparticles in a comparative example supporting the same physiologically active substance was calculated on the basis of the following equation.

[Math. 14]

$$[\text{Math.14}]$$

$$\text{Support amount ratio} = \frac{\text{HPLC peak area in example}}{\text{HPLC peak area in comparative example}}$$

HPLC measurement was carried out as follows. After weighing 25 mg each of the microparticles in the examples and the comparative examples, the microparticles were respectively put in 50-mL volumetric flasks. Each type of microparticles was completely dissolved in 5 mL of ethanol added, and the solution was diluted with deionized water added and passed through a 0.45-$\mu$m filter to provide an HPLC sample. The sample was measured under the same conditions as for Test Example 5.

[Table 7]

[Table7]

| Physiologically active substance | Support amount ratio (= example/comparative example) |
|---|---|
| Salicylic acid | 1.6 |
| Theophylline | 1.2 |
| Quinine hydrochloride dihydrate | 1.8 |
| Thiamine hydrochloride | 10.7 |
| Acetaminophen | 1.4 |
| Ibuprofen | 1.2 |

Table 7 shows that the supported amount ratios were larger than 1 for all the physiologically active substances and that the porous structure was effective in supporting the physiologically active substances.

[Example 16]

A 50 vol% aqueous solution of ethanol was obtained by mixing 100 mL of deionized water and 100 mL of ethanol. Using a 50-mL volumetric flask, 1 g of anhydrous caffeine (manufactured by Kanto Chemical Co., Inc., extra pure) was dissolved in the 50 vol% aqueous solution of ethanol to provide a 20 mg/mL anhydrous caffeine solution. To a piece of filter paper, 10 g of the microparticles in Example 6 was transferred, the 20 mg/mL anhydrous caffeine solution was added, and suction filtration was carried out. The solid matter on the filter paper was dried at 50°C for 4 hours in a vacuum dryer. Observation of the resulting dried product at a magnification of 500 times under an FE-SEM showed that crude crystals of caffeine were not mixed on the surface or the like of the microparticles and that the microparticles had similar appearance to the microparticles in Example 6. An HPLC sample was prepared from the resulting microparticles through substantially the same operations as in Test Example 8, and the amount of anhydrous caffeine supported on the microparticles was measured under the following conditions.

<HPLC Conditions>

Mobile phase: 20 mM aqueous solution of potassium dihydrogenphosphate/acetonitrile = 90/10 (v/v)
Column: L-Column2 ODS (manufactured by Chemicals Evaluation and Research Institute, Japan; 3.0 mm in diameter × 250 mm)
Column temperature: 40°C
Detection Wavelength: 264 nm

The supporting rate (%) of anhydrous caffeine calculated from the equation below using the obtained supported amount of anhydrous caffeine was 0.3%. This result showed that particles for preparation in which the porous ethyl cellulose microparticles supported anhydrous caffeine were obtained.

[Math. 15]

[Math.15]

$$\text{Supporting rate} = \frac{\text{Supported amount of physiologically active substance measured by HPLC}}{\text{Weight of microparticles used for supporting}} \times 100$$

[Example 17]

(Example of Supporting of Physiologically Active Substance on Microparticles using Mortar (Agitation Granulation))

Using a 10-mL volumetric flask, 5 g of sodium salicylate was diluted with deionized water to provide a 0.5 g/mL aqueous solution of sodium salicylate. To a mortar, 1 g of the microparticles in Example 6 was transferred, 0.7 g of the 0.5 g/mL aqueous solution of sodium salicylate was added and mixed in small amounts, and the resulting microparticles were dried at 50°C for 1 hour in a vacuum dryer. Observation of the resulting dried product at a magnification of 500 times under an FE-SEM showed that crude crystals of sodium salicylate were not mixed on the surface or the like of the microparticles and that the microparticles had similar appearance to the microparticles in Example 6. An HPLC sample was prepared from the resulting microparticles through substantially the same operations as in Test Example 8, and the amount of sodium salicylate supported on the microparticles was measured under the HPLC conditions in Example 16. The supporting rate calculated from the above equation was 28%, which indicated that particles for preparation in which the porous ethyl cellulose microparticles supported sodium salicylate were obtained.

[Example 18]

(Example of Supporting of Physiologically Active Substance on Microparticles by Fluidized Bed Granulation)

In 100 g of deionized water, 2.5 g of sodium salicylate and 0.1 g of sodium lauryl sulfate (manufactured by Nikko Chemicals Co., Ltd.) were dissolved. A micro fluidized-bed coater (manufactured by Dalton Corporation) was charged with 10 g of the porous microparticles in Example 6, and the aqueous solution of sodium salicylate and sodium lauryl sulfate was sprayed under conditions of an intake temperature of 75°C, a speed of the spray liquid of 0.3 g/min, and an exhaust temperature of about 33°C. Observation of the resulting dried product at a magnification of 500 times under an FE-SEM showed that crude crystals of sodium salicylate or sodium lauryl sulfate were not mixed on the surface or the like of the microparticles and that the microparticles had similar appearance to the microparticles in Example 6. An HPLC sample was prepared from the resulting microparticles through substantially the same operations as in Test Example 8, and the amount of sodium salicylate supported on the microparticles was measured under the conditions in Example 16. The supporting rate calculated from the above equation was 12%, which indicated that particles for preparation in which the porous ethyl cellulose microparticles supported sodium salicylate were obtained.

[Example 19]

(Example of Layering Coating of Sustained-Release Layer on Surface of Particles for Preparation)

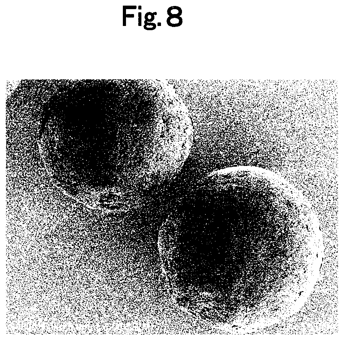

A suspension of sustained-release agents was prepared by mixing 43.5 parts by mass of an enteric methacrylate copolymer ("Eudragit (registered trademark)" L30D-55 manufactured by Evonik Industries AG), 2.6 parts by mass of poly(ethylene glycol) (PEG-6000 manufactured by Nippon Oil & Fats Co., Ltd.), 1.7 parts by mass of triethyl citrate ("Citroflex (registered trademark)" 2 manufactured by Pfizer Inc.), and 152 parts by mass of distilled water. A micro fluidized-bed coater (manufactured by Dalton Corporation) was charged with 10 g of the particles for preparation in Example 9, and the suspension of sustained-release agents was sprayed under conditions of an intake temperature of 28°C, a speed of the spray liquid of 0.1 g/min, and an exhaust temperature of about 26°C. Talc (Crown Talc Official Drug PP manufactured by Matsumura Sangyo K.K.) was timely added in the form of powder according to the fluidity state. The particles were collected when 70% of the particles for preparation in Example 9 were covered, and the coating was formed at 40°C and a relative humidity of 75% RH for 16 hours. Particles for preparation having a sustained-release layer in which the particles for preparation in Example 9 were coated with a controlled-release layer were thus obtained.
Observation of the particles for preparation in Example 19 under an FE-SEM (at a magnification of 600 times) shown in Fig. 8 showed that the surface was smooth and that the coating of the sustained-release layer was layered.

[Example 20]

(Example of Surface Modification of Particles for Preparation)

A mixture of 250 mg of the particles for preparation in Example 16 and 50 mg of talc was transferred to a glass vial, and its upper portion was covered with gauze. To a 25-mL screw-cap vial, 5 mL of ethanol was added. The glass vial was put in the screw-cap vial such that the glass vial did not have contact with ethanol, and the screw-cap vial was tightly sealed. The screw-cap vial was allowed to stand still at 55°C for 48 hours. Surface-modified particles for preparation in which the surface of the particles for preparation in Example 16 was modified with ethanol vapor were thus obtained.

[Example 21]

(Example of Surface Modification of Particles for Preparation)

A mixture of 110 mg of the particles for preparation in Example 12 and 40 mg of talc was transferred to a glass vial, and its upper portion was covered with gauze. To a 25-mL screw-cap vial, 5 mL of ethanol was added. The glass vial was put in the screw-cap vial such that the glass vial did not have contact with ethanol, and the screw-cap vial was tightly sealed. The screw-cap vial was allowed to stand still at 60°C for 1 hour. Particles for preparation taste-masked by modifying the surface of the particles for preparation in Example 12 with ethanol vapor were thus obtained.

[Example 22]

(Example of Formation of Sustained-Release Layer by Filling Fine Pores of Particles for Preparation with Hydrophobic Substance)

Heating and melting of 70 parts by mass of lauric acid (NAA-122 manufactured by Nippon Oil & Fats Co., Ltd.) and 30 parts by mass of poly(ethylene glycol) (PEG-6000) was carried out. To a mortar, 1 g of the particles for preparation in Example 9 was transferred, and 0.5 g of the melt was mixed while heating the melt. The resulting particles were sifted through a sieve with an opening of 355 $\mu$m to mix the hydrophobic substance. Particles for preparation having a sustained-release layer in which the particles for preparation in Example 9 were covered with the sustained-release layer were thus obtained.

[Example 23]

(Production Example of Tablet)

A mixture of 50 parts by mass of the particles for preparation in Example 9 and 50 parts by mass of hydroxypropyl cellulose (HPC-L FP manufactured by Nippon Soda Co., Ltd.) was tableted under a pressure of 24 kN/cm$^2$ using an IR tableting machine (manufactured by Riken Seiki Co., Ltd.) to produce a tablet having a total weight of 100 mg and a planar shape with a diameter of 6 mm. The tablet hardness of the resulting tablet was measured using a load-cell-type tablet hardness tester (Portable Checker PC-30 manufactured by Okada Seiko Co., Ltd.). The average of 3 measurement values was 112 N, which is a sufficient hardness in handling of practical hardness.

[Example 24]

(Production Example of Orally Disintegrating Tablet)

A mixture of 50 parts by mass of the surface-modified particles for preparation in Example 20, 10 parts by mass of crystalline cellulose (manufactured by Asahi Kasei Chemicals Corporation, "CEOLUS (registered trademark)" KG-1000, manufactured by Asahi Kasei Corporation), 40 parts by mass of mannitol ("PEARLITOL (registered trademark)" 200SD manufactured by Roquette Japan K.K.), 9 parts by mass of crospovidone ("Kollidon (registered trademark)" CL manufactured by BASF SE), and 1 part by mass of sodium stearyl fumarate (manufactured by K.K. Nissei Kagaku Kogyosho) was tableted under a pressure of 15 kN/cm$^2$ using an IR tableting machine to produce a tablet having a total weight of 110 mg and a planar shape with a diameter of 7 mm. The tablet hardness of the resulting tablet was measured using a load-cell-type tablet hardness tester (Portable Checker PC-30 manufactured by Okada Seiko Co., Ltd.). The average of 3 measurement values was 45 N. Also, the time elapsed before the tablet that was kept in the mouth disintegrated without taking water or chewing the tablet was measured as the oral disintegration time. The average of 3 measurement values was 10 seconds, which is an appropriate property as an orally disintegrating tablet.

[Test Example 9; Dissolution Test]

Dissolution tests on the particles in Examples 16, 19, 20, 23, and 24 and anhydrous caffeine particles were carried out according to Method 2 (Puddle Method) of the Dissolution Test in The Japanese Pharmacopoeia under the following conditions.

Test solution: distilled water containing 0.1% of Tween 80

Artificial gastric juice containing 0.1% of Tween 80 (solution JP1)
Artificial intestinal juice containing 0.1% of Tween 80 (solution JP2)

Rotation rate of puddle: 50 rpm

The sampled solution was directly used as an HPLC sample.

<HPLC Conditions>

Mobile phase: 20 mM aqueous solution of potassium dihydrogenphosphate/acetonitrile = 90/10 (v/v)
Column: L-Column2 ODS (manufactured by Chemicals Evaluation and Research Institute, Japan; 3.0 mm in diameter × 250 mm)
Column temperature: 40°C
Detection Wavelength: 264 nm

The dissolution rates (%) of the physiologically active substances were calculated from the equation below using the results of HPLC. The "HPLC peak area when 100% of the physiologically active substance has been eluted" in the equation was obtained by carrying out a measurement through substantially the same operations as in the method in Test Example 8 under the above conditions on a solution obtained by completely dissolving each type of the microparticles in ethanol, diluting the solution with deionized water, and passing the solution through a 0.45-$\mu$m filter.
[Math. 16]

[Math.16]

Dissolution rate

$$= \frac{\text{HPLC peak area of physiologically active substance in test solution}}{\text{HPLC peak area when 100\% of physiologically active substance has been eluted}}$$

$\times\, 100$

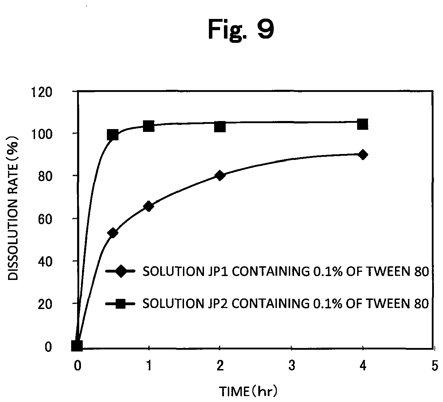

Fig. 9 shows the results of dissolution tests on the particles for preparation having a sustained-release layer in Example 19. As shown in Fig. 9, the porous ethyl cellulose microparticles on which the coating of the enteric sustained-release layer was layered exhibited such a desired dissolution rate that elution of the physiologically active substance was notably suppressed in the artificial gastric juice (solution JP1) and that the physiologically active substance was rapidly released in the artificial intestinal juice (solution JP2).

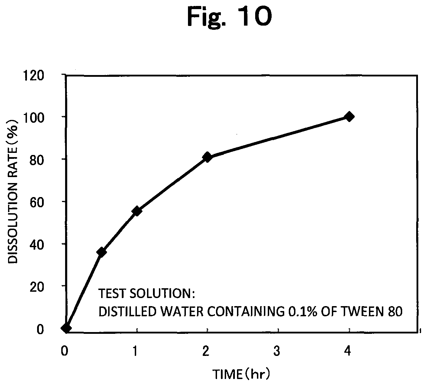

Fig. 10 shows the result of a dissolution test on the tablet in Example 23. As shown in Fig. 10, elution of the physiologically active substance from the tablet containing the porous ethyl cellulose microparticles showed the sustained-release property.

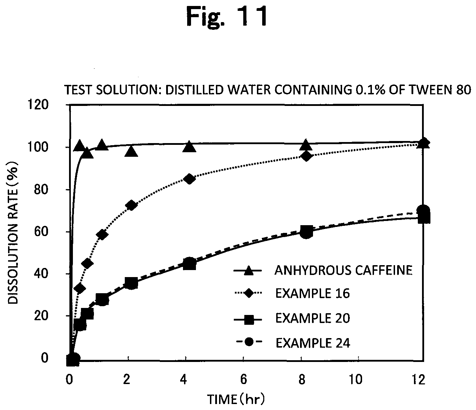

Fig. 11 shows the results of dissolution tests on the particles for preparation supporting anhydrous caffeine in Example 16, the particles for preparation in Example 20, the orally disintegrating tablet in Example 24, and anhydrous caffeine (manufactured by Kanto Chemical Co., Inc., extra pure) particles. As shown in Fig. 11, elution of the physiologically active substance from the particles and the orally disintegrating tablet showed the sustained-release property compared with the naked anhydrous caffeine particles. Furthermore, it was shown that the particles for preparation in Example 16 could release 100% of the physiologically active substance. Also, the particles for preparation in Example 20 obtained by surface-modifying the particles for preparation in Example 16 had a lower release rate than the release rate of the particles for preparation in Example 16, which showed that the sustained-release property could be imparted. Furthermore, the orally disintegrating tablet in Example 24 obtained by compressing the particles for preparation in Example 20 at an ordinary tableting pressure had a dissolution rate close to the dissolution rate of the particles for preparation in Example 20, which showed that the sustained-release preparation that was stable against compression pressure was obtained.

[Test Example 10; Taste-Masking Effect]

Tastes of the ethyl cellulose particles supporting quinine hydrochloride dihydrate in Comparative Example 8 and the particles for preparation supporting quinine hydrochloride dihydrate in Examples 12 and 21 were tested. Three healthy testers kept each type of the particles in their mouths for one minute and evaluated the bitter tastes felt on the basis of the scores below. Table 8 shows the average bitter taste scores of the scores given by the three testers. As shown in Table 8, the particles for preparation in Examples 12 and 21 had better masking effects of bitter tastes than in Comparative Example 8.

<Masking Scores of Bitter Taste>

    1. No bitter taste
    2. Almost no bitter taste
    3. Slightly bitter
    4. Strongly bitter
    5. Intensely bitter

[Table 8]

[Table8]

|  | Quinine hydrochloride dihydrate | Comparative Example 8 | Example 12 | Example 21 |
|---|---|---|---|---|
| Average bitter taste score | 4.7 | 3.3 | 2.0 | 1.0 |

[Test Example 11; Dissolution Test Regarding Selective Supporting of Emulsion]

In 18.5 g of ethanol, 1.5 g of α-tocopherol was dissolved. Test Emulsion 1 was obtained by collecting 2 mL of the solution, adding 2 mL of water, and stirring the mixture with a stirrer. On an ultrafiltration membrane for a centrifuge ("Nanosep 100K Omega (registered trademark)" manufactured by Pall Corporation), about 44 mg of the microparticles in Example 6 were gently put, 400 μL of Test Emulsion 1 was added, and the mixture was centrifuged. The filtrate was diluted 10 times with an acetonitrile/methanol = 6/4 (v/v) mixed solution to provide an HPLC sample.

<HPLC Conditions>

Mobile phase: acetonitrile/methanol = 6/4 (v/v)
Column: YMC-Pack Pro C18 (manufactured by YMC Co., Ltd.; 4.6 mm in diameter × 250 mm)
Column temperature: 35°C
Detection wavelength: 210 nm

The adsorption rate (%) of α-tocopherol on the porous microparticles in Example 6 was calculated from the equation below using peaks obtained as a result of HPLC analysis of the HPLC sample.

[Math. 17]

$$[\text{Math.17}]$$

$$\text{Adsorption rate} = 100 - \frac{\text{HPLC peak area of filtrate}}{\text{HPLC peak area of Test Emulsion 1}} \times 100$$

The obtained adsorption rate of α-tocopherol on the microparticles in Example 6 was 99.8%, which indicated that most part of α-tocopherol in the filtrate was adsorbed and supported on the microparticles in Example 6.

[Example 25]

A 50 vol% aqueous solution of ethanol was obtained by mixing 100 mL of deionized water and 100 mL of ethanol. Using a 5-mL volumetric flask, 20 mg of beraprost sodium was diluted with the 50 vol% aqueous solution of ethanol to provide a 4 mg/mL beraprost sodium solution. To a mortar, 3.2 g of the microparticles in Example 6 was transferred, and 1 mL of a 4 g/mL beraprost sodium solution was added and mixed in small amounts. The resulting microparticles were dried at 50°C for 2 hours in a vacuum dryer to provide particles for preparation in which the porous ethyl cellulose microparticles supported beraprost sodium. Furthermore, 0.8 g of talc was mixed to prevent adhesion between the particles for preparation to provide a mechanical mixture of the particles for preparation and talc.

[Example 26]

Particles for preparation in which the porous ethyl cellulose microparticles supported beraprost sodium were obtained in substantially the same manner as in Example 26 except that the solvent was changed from the 50 vol% aqueous solution of ethanol used in Example 25 to a 40 vol% aqueous solution of ethanol. Furthermore, 0.8 g of talc was mixed to prevent adhesion between the particles for preparation to provide a mechanical mixture of the particles for preparation and talc.

[Example 27]

Particles for preparation in which the porous ethyl cellulose microparticles supported beraprost sodium were obtained in substantially the same manner as in Example 26 except that the solvent was changed from the 50 vol% aqueous solution of ethanol used in Example 25 to a 30 vol% aqueous solution of ethanol. Furthermore, 0.8 g of talc was mixed to prevent adhesion between the particles for preparation to provide a mechanical mixture of the particles for preparation and talc.

[Comparative Example 12]

Crystalline cellulose supporting beraprost sodium was obtained in substantially the same manner as in Example 25 except that crystalline cellulose ("Celphere (registered trademark)" CP102 manufactured by Asahi Kasei Corporation) was used instead of the porous ethyl cellulose microparticles in Example 6. Furthermore, 0.8 g of talc was mixed to prevent adhesion between the particles for preparation to provide a mechanical mixture of the particles for preparation and talc.

[Comparative Example 13]

Crystalline cellulose supporting beraprost sodium was obtained in substantially the same manner as in Example 26 except that crystalline cellulose ("Celphere (registered trademark)" CP102 manufactured by Asahi Kasei Corporation) was used instead of the ethyl cellulose particles in Example 6. Furthermore, 0.8 g of talc was mixed to prevent adhesion between the particles for preparation to provide a mechanical mixture of the particles for preparation and talc.

[Test example 12; Dissolution Test for Beraprost Sodium]

Dissolution tests on Examples 25 to 27 and Comparative Examples 12 and 13 were carried out under the following conditions.

Test solution: distilled water containing 0.1% of Tween 80
Rotation rate of puddle: 50 rpm

The sampled solution and methanol were mixed in equal proportions to provide an HPLC sample.

<HPLC Conditions>

Mobile phase: acetic acid/distilled water/methanol = 1/350/650 (v/v)
Column: YMC-Pack ODS-AM (manufactured by YMC Co., Ltd.; 3.0 mm in diameter $\times$ 150 mm)
Column temperature: 40°C
Detection wavelengths: excitation wavelength 285 nm, fluorescence wavelength 614 nm

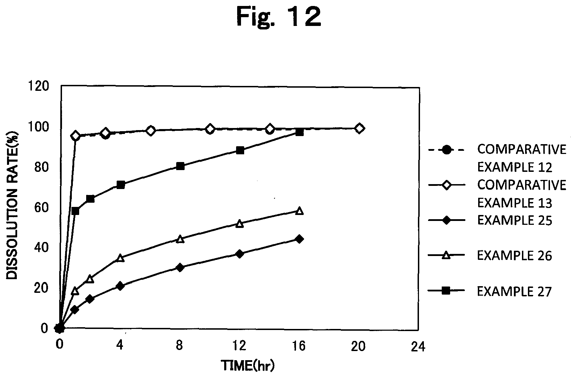

Fig. 12 shows the results of dissolution tests for beraprost sodium on the mechanical mixtures of the particles for preparation and talc in Example 25 to Example 27 and the mechanical mixtures of crystalline cellulose and talc in Comparative Example 12 and Comparative Example 13. Fig. 12 showed that a preferable sustained-release property could be obtained even in the case where beraprost sodium was used as a physiologically active substance supported on the ether cellulose derivative microparticles.

It was also shown that the sustained-release property of the particles for preparation could be adjusted by selecting a solvent used for causing the ether cellulose derivative microparticles to support the physiologically active substance. Specifically, comparison of the elution rate in Example 25, which employed the 50 vol% aqueous solution of ethanol as the solvent for causing the physiologically active substance to be supported, with the elution rates in Example 26, which employed the 40 vol% aqueous solution of ethanol, and in Example 27, which employed the 30 vol% aqueous solution of ethanol, showed that the elution rate of the physiologically active substance became faster as the proportion of ethanol, which is a solvent that dissolves the ether cellulose derivative microparticles, decreased. Accordingly, it was shown that the sustained-release property could be adjusted by selecting a solvent for supporting, which includes changing the concentration of the solvent for supporting. In addition, since the crystalline cellulose particles in Comparative Example 12 and Comparative Example 13 are insoluble in both ethanol and water, the particles did not exhibit the sustained-release property regardless of the solvents for supporting and exhibited rapid release.

[Examples 28 to 30]

To a glass vial, 2 g of the mechanical mixture obtained in Example 25 was transferred, and its upper portion was covered with gauze. To a screw-cap vial, 5 mL of ethanol was added. The glass vial was put in the screw-cap vial such that the glass vial did not have contact with ethanol, and the screw-cap vial was tightly sealed. The screw-cap vial was allowed to stand still at 60°C, and particles for preparation surface-modified with ethanol vapor for 30 minutes, 1 hour, or 2 hours were obtained. The particles surface-modified for 30 minutes correspond to Example 28, the particles surface-modified for 1 hour correspond to Example 29, and the particles surface-modified for 2 hours correspond to Example 30.

[Test Example 13; Dissolution Test after Surface Modification]

Dissolution tests on Example 25 and Examples 28 to 30 were carried out under the same conditions as in Test Example 12.

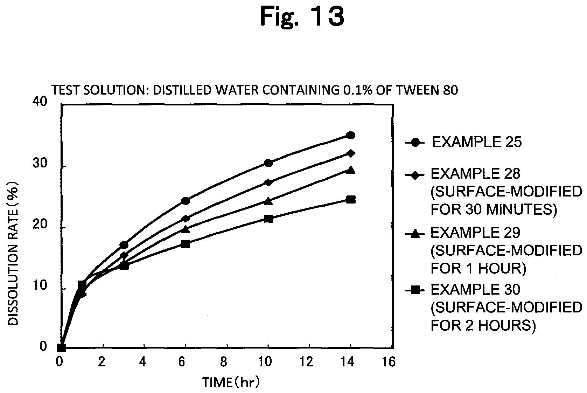

Fig. 13 shows the results of dissolution tests on the mechanical mixture of the particles for preparation and talc in Example 25 and mechanical mixtures of the particles for preparation and talc for which different time periods of surface modification were employed (Examples 28 to 30). The release rate decreased depending on the time period of surface modification for the particles for preparation in Examples 28 to 30, which showed that the sustained-release property could be controlled.

[Example 31]

In 18.5 g of ethanol, 1.5 g of $\alpha$-tocopherol was dissolved. Test Emulsion 1 was obtained by collecting 2 mL of the solution, adding 2 mL of water, and stirring the mixture with a stirrer. To a mortar, about 1.2 g of the microparticles in Example 6 was transferred, and the microparticles were mixed with 1 mL of Test Emulsion 1. The mixture was dried at room temperature for 2 hours in a vacuum dryer to provide particles for preparation in which the porous ethyl cellulose microparticles supported $\alpha$-tocopherol at about 3%.

[Example 32]

About 80 mg of talc was mixed with about 320 mg of the particles for preparation obtained in Example 31 to prevent adhesion between the particles for preparation to provide a mechanical mixture of the particles for preparation and talc. The mixture was transferred to a glass vial, and its upper portion was covered with gauze. To a screw-cap vial, 5 mL of ethanol was added. The glass vial was put in the screw-cap vial such that the glass vial did not have contact with ethanol, and the screw-cap vial was tightly sealed. The screw-cap vial was allowed to stand still at 60°C for 2 hours to provide a mechanical mixture of the particles for preparation and talc obtained by surface-modifying the particles for preparation in Example 31.

[Example 33]

In 6 mL of ethanol, about 3 g of $\alpha$-tocopherol was dissolved. Test Emulsion 2 was obtained by collecting 1 mL of the solution, adding 1 mL of water, and vigorously stirring the mixture with a stirrer. About 1.2 g of the microparticles in Example 6 were transferred to a mortar and mixed with 1 mL of Test Emulsion 2. The mixture was dried at room temperature for 2 hours in a vacuum dryer to provide particles for preparation in which the porous ethyl cellulose microparticles supported $\alpha$-tocopherol at about 10%.

[Example 34]

About 80 mg of talc was mixed with about 320 mg of the particles for preparation obtained in Example 33 to prevent adhesion between the particles to provide a mechanical mixture of the particles for preparation and talc. The mixture was transferred to a glass vial, and its upper portion was covered with gauze. To a screw-cap vial, 5 mL of ethanol was added. The glass vial was put in the screw-cap vial such that the glass vial did not have contact with ethanol, and the screw-cap vial was tightly sealed. The screw-cap vial was allowed to stand still at 60°C for 2 hours to provide a mechanical mixture of the particles for preparation and talc obtained by surface-modifying the particles for preparation in Example 33.

[Test Example 14; Dissolution Test for $\alpha$-Tocopherol]

Dissolution tests on Examples 31 to 34 were carried out under the following conditions.
Test solution: artificial intestinal juice containing 1% of Tween 80 (solution JP2)
Rotation rate of puddle: 50 rpm

<HPLC Conditions>

Measurement on the sampled solution was carried out under the same conditions as for Test Example 9.

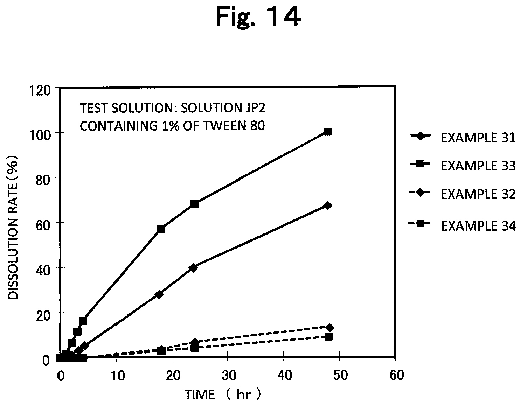

Fig. 14 shows the results of dissolution tests for $\alpha$-tocopherol on the particles for preparation in Example 31, the mechanical mixture of the particles for preparation and talc in Example 32, the particles for preparation in Example 33, and the mechanical mixture of the particles for preparation and talc having been subjected to surface modification in Example 34. Fig. 14 showed that such a preferable sustained-release property could be obtained that $\alpha$-tocopherol was eluted from the microparticles over time in the case where $\alpha$-tocopherol was supported on the porous ether cellulose derivative microparticles. It was also shown that surface modification further reduced the elution rate and that the drug dissolution rate was kept equal to or less than 20% even after 48 hours, which indicated improvement in the sustained-release performance of the particles for preparation.

**Claims**

1. An ether cellulose derivative microparticle having:

   an average particle diameter of 1 to 1,000 $\mu$m;
   a linseed oil absorption of 50 to 1,000 mL/100 g; and
   an average surface pore size of 0.05 to 5 $\mu$m.

2. The ether cellulose derivative microparticle according to claim 1, the ether cellulose derivative microparticle having a pore volume calculated by a mercury intrusion method of 0.05 to 5 cm$^3$/g.

3. The ether cellulose derivative microparticle according to either claim 1 or claim 2, the ether cellulose derivative microparticle having a sphericity of equal to or more than 80.

4. The ether cellulose derivative microparticle according to any one of claims 1 to 3, the ether cellulose derivative microparticle having a particle diameter distribution index of 1 to 3.

5. The ether cellulose derivative microparticle according to any one of claims 1 to 4, the ether cellulose derivative microparticle having a bulk density of 0.05 to 1.0 g/mL.

6. The ether cellulose derivative microparticle according to any one of claims 1 to 5, the ether cellulose derivative microparticle having a degree of crystallinity of equal to or more than 1%.

7. The ether cellulose derivative microparticle according to any one of claims 1 to 6, wherein an ether cellulose derivative constituting the ether cellulose derivative microparticle comprises an alkyl cellulose.

8. The ether cellulose derivative microparticle according to claim 7, wherein the alkyl cellulose comprises ethyl cellulose.

9. A dispersion comprising the ether cellulose derivative microparticle according to any one of claims 1 to 8.

10. A method for producing the ether cellulose derivative microparticle according to any one of claims 1 to 8, the method comprising, in a system where phase separation into two phases occurs when an ether cellulose derivative (A), a polymer (B) different from the ether cellulose derivative (A), and an alcohol solvent (C) are mixed together, the two phases comprising a solution phase mainly comprising the ether cellulose derivative (A) and a solution phase mainly comprising the polymer (B), the two separated phases comprising approximately same solvent:

    forming an emulsion of the ether cellulose derivative (A), the polymer (B), and the alcohol solvent (C); and
    bringing the emulsion into contact with a poor solvent (D) for the ether cellulose derivative (A) to precipitate the ether cellulose derivative microparticle.

11. The method for producing the ether cellulose derivative microparticle according to claim 10, wherein the ether cellulose derivative (A) comprises an ether cellulose derivative having a degree of crystallinity of equal to or more than 2%.

12. A composite microparticle comprising:

the ether cellulose derivative microparticle according to any one of claims 1 to 8; and
an active ingredient.

13. The composite microparticle according to claim 12, wherein the active ingredient comprises at least one selected from a physiologically active substance, a perfume, a sweetener, an acidulant, an antioxidant, a preservative, a disinfectant, a coloring agent, an agricultural chemical, a fertilizer, a repellent, an attractant, a fungicide, a sterilant, a germicide, an antimicrobial agent, an antibacterial agent, a preservative agent, an antiseptic agent, a deodorizer, and a lubricant.

14. The composite microparticle according to claim 12 or 13, the composite microparticle further comprising on a surface a sustained-release layer configured to suppress release of the active ingredient from the composite microparticle.

15. A preparation comprising the composite microparticle according to any one of claims 12 to 14.

## Fig. 1

# Fig. 2

Fig.3

# Fig. 4

## Fig. 5

# Fig. 6

# Fig.7

Fig. 8

# Fig. 9

# Fig. 10

# Fig. 11

TEST SOLUTION: DISTILLED WATER CONTAINING 0.1% OF TWEEN 80

# Fig. 12

# Fig. 13

TEST SOLUTION: DISTILLED WATER CONTAINING 0.1% OF TWEEN 80

EXAMPLE 25

EXAMPLE 28
(SURFACE-MODIFIED
FOR 30 MINUTES)

EXAMPLE 29
(SURFACE-MODIFIED
FOR 1 HOUR)

EXAMPLE 30
(SURFACE-MODIFIED
FOR 2 HOURS)

# Fig. 14

**INTERNATIONAL SEARCH REPORT**

International application No.

PCT/JP2016/081748

### A. CLASSIFICATION OF SUBJECT MATTER

*C08B11/02*(2006.01)i

According to International Patent Classification (IPC) or to both national classification and IPC

### B. FIELDS SEARCHED

Minimum documentation searched (classification system followed by classification symbols)

C08B11/02, A61K47/38

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>A | JP 01-275601 A (Kaneka Corp.),<br>06 November 1989 (06.11.1989),<br>claims; page 2, lower left column, line 5 to upper right column, line 7; page 3, upper left column, lines 5 to 18; page 3, lower right column, line 8 to page 4, upper right column, line 9<br>(Family: none) | 1-9,12-15<br>10-11 |
| X<br>A | JP 10-237101 A (Daicel Chemical Industries, Ltd.),<br>08 September 1998 (08.09.1998),<br>claims<br>(Family: none) | 1-9,12-15<br>10-11 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 December 2016 (22.12.16) | 10 January 2017 (10.01.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (January 2015)

**INTERNATIONAL SEARCH REPORT**

| International application No. |
| --- |
| PCT/JP2016/081748 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | WO 2012/033223 A1  (Kaneka Corp.),<br>15 March 2012 (15.03.2012),<br>& US 2013/0172538 A1    & EP 2626381 A1 | 1-15 |
| A | JP 11-158202 A  (Kaneka Corp.),<br>15 June 1999 (15.06.1999),<br>& WO 1998/030620 A1     & US 2003/0012941 A1<br>& EP 955332 A1 | 1-15 |
| P,X<br>P,A | EP 3011956 A1  (SHIN-ETSU CHEMICAL CO., LTD.),<br>27 April 2016 (27.04.2016),<br>claims<br>& US 2016/0113875 A1     & JP 2016-84469 A<br>& CN 105542016 A            & KR 10-2016-0047992 A | 1-9,12-15<br>10-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015214372 A
- JP 2016020548 A
- JP 2016020749 A
- JP 2016108946 A
- JP S6383144 A
- WO 2012033223 A
- WO 2009123148 A
- JP 2003252903 A
- JP H2235944 A

**Non-patent literature cited in the description**

- **YAMAMOTO ; HIDEKI.** SP-chi Kiso·Ouyou To Keisanhouhou. Johokiko Co., Ltd, 31 March 2005
- **BRAND.** J. Polymer Handbook. Wiley, 1998